# EUROPEAN PATENT SPECIFICATION

(11) **EP 2 294 078 B1**
(45) Date of publication and mention of the grant of the patent: **06.01.2016**
(21) Application number: 09761709.6
(22) Date of filing: 09.06.2009
(51) Int. Cl.: C07K 14/175, A61K 39/12

(54) **RIFT VALLEY FEVER VIRUS-LIKE PARTICLES AND THEIR USE FOR IMMUNIZATION AND AS TEST SYSTEM**
RIFT-VALLEY-FIEBERVIRUSARTIGE PARTIKEL UND IHRE VERWENDUNG ZUR IMMUNISIERUNG SOWIE TESTSYSTEM DAFÜR
PARTICULES PSEUDOVIRALES DU VIRUS DE LA FIÈVRE DE LA VALLÉE DU RIFT, ET UTILISATION DE CELLES-CI POUR L'IMMUNISATION ET COMME SYSTÈME D'ESSAI

(30) Priority: 10.06.2008 EP 08010548
(43) Date of publication of application: 16.03.2011
(73) Proprietor: Universitätsklinikum Freiburg, 79106 Freiburg (DE)
(72) Inventor: WEBER, Friedemann, 35041 Marburg-Michelbach (DE); HABJAN, Matthias, 9016 St. Gallen (CH); SPIEGEL, Martin, 37075 Göttingen (DE); PENSKI, Nicola, 9016 St. Gallen (CH)
(74) Representative: Keller, Günter
(86) International application number: PCT/EP2009/057078
(87) International publication number: WO 2009/150146

(56) References cited:
- WO-A-2007/104979
- OVERBY ANNA K ET AL: "Generation and analysis of infectious virus-like particles of Uukuniemi virus (Bunyavifidae): a useful system for studying bunyaviral packaging and budding" JOURNAL OF VIROLOGY, vol. 80, no. 21, November 2006 (2006-11), pages 10428-10435, XP002499943 ISSN: 0022-538X
- LIU LI ET AL: "Rift Valley fever virus structural proteins: expression, characterization and assembly of recombinant proteins." VIROLOGY JOURNAL 2008, vol. 5, 2008, page 82, XP002499944 ISSN: 1743-422X
- KOHL ALAIN ET AL: "Genetic elements regulating packaging of the Bunyamwera orthobunyavirus genome" JOURNAL OF GENERAL VIROLOGY, vol. 87, no. Part 1, January 2006 (2006-01), pages 177-187, XP002499945 ISSN: 0022-1317
- HABJAN M ET AL: "Efficient production of Rift Valley fever virus-like particles: The antiviral protein MxA can inhibit primary transcription of bunyaviruses" VIROLOGY, ACADEMIC PRESS,ORLANDO, US, vol. 385, no. 2, 15 March 2009 (2009-03-15), pages 400-408, XP025989972 ISSN: 0042-6822 [retrieved on 2009-01-19]
- NASLUND J ET AL: "Vaccination with virus-like particles protects mice from lethal infection of Rift Valley Fever Virus" VIROLOGY, ACADEMIC PRESS,ORLANDO, US, vol. 385, no. 2, 15 March 2009 (2009-03-15), pages 409-415, XP025989973 ISSN: 0042-6822 [retrieved on 2009-01-20]

## Description

Rift Valley fever Virus (RVFV) is a serious emerging pathogen affecting humans and livestock in sub-Saharan Africa, Egypt, Yemen, and Saudi Arabia. Since the first description of an outbreak in Kenya in 1931, there have been recurrent epidemics, killing thousands of animals, hundreds of humans, and causing significant economic losses. To animals, RVFV is mainly transmitted by mosquitoes, but humans are often infected by close contact with infected animals' material. A wide array of symptoms is connected with the human disease, ranging from an uncomplicated acute febrile illness to retinitis, hepatitis, meningoencephalitis, severe hemorrhagic disease, and death. The severity of RVFV zoonosis as well as the capability to cause major epidemics in livestock and humans have prompted authorities to list RVFV as a notifiable disease and a potential biological weapon.

RVFV belongs to the genus Phlebovirus, family *Bunyaviridae.* Bunyaviruses are enveloped and have a tri-segmented single-stranded RNA genome of negative or ambisense polarity, replicate in the cytoplasm, and bud into the Golgi apparatus. They encode five common structural proteins:
the viral polymerase (L) on the large (L) segment,
two glycoproteins (Gn and Gc) and the 78kD protein on the medium (M) segment, and
the viral nucleocapsid protein (N) on the smallest (S) segment.

RVFV additionally expresses two nonstructural proteins, encoded on the M segment (termed NSm) and the S segment (termed NSs). These accessory proteins are dispensable for viral multiplication in cell culture, but play important roles for pathogenesis *in vivo.* In particular, the NSm and 78kD proteins were found to enhance intrahost viral spread, whereas NSs is important to suppress the antiviral interferon system.

The general features of RVFV transcription and replication are similar to those of other negative-stranded RNA viruses. The viral genomic RNA (vRNA) segments contain untranslated regions (UTRs) on both the 3' and the 5' ends that serve as promoters for replication of the segment and transcription of the encoded reading frames. The vRNAs are encapsidated by N protein and associate with L protein both intracellularly and in the virion, and only these ribonucleoprotein particles (RNPs) are functional templates for mRNA synthesis and RNA replication by the viral polymerase.

After Gn/Gc-mediated entry into the cytoplasm, the negative-sense vRNAs of RVFV are transcribed into mRNAs by the RNP-associated L and N proteins ("primary transcription"). The products of these immediate early mRNAs then provide the machinery for replication of the genome and further mRNA synthesis ("secondary transcription"), and later drive the packaging of newly assembled RNPs into budding particles. A specific feature of bunyaviruses is that their mRNA synthesis is strictly dependent on host translation. Most likely, ongoing translation prevents premature termination of transcription by secondary structures building up on the nascent mRNA.

The infection cycle of bunyaviruses is strongly inhibited by the interferon-induced MxA protein of humans. The cytoplasmatically located MxA is capable of binding recombinant N protein of RVFV and several other bunyaviruses, and to sequester it to perinuclear complexes. It remained unclear, however, whether MxA solely cuts bunyaviruses off the supply of N protein, or whether it also affects assembled RNPs and inhibits their transcriptional activity.

Recently, reverse genetic systems to rescue infectious RVFV particles entirely from cloned cDNA plasmids have been developed (Ikegami, Journal of Virology [May 2005], pp 5606-5615; Gerrard et al., Virology 359, 459-65 [2007]). These systems allow the targeted manipulation of the viral genome and proved to be extremely useful to demonstrate the contribution of the NSs and the NSm proteins to viral pathogenesis and to identify transcriptional termination signals.

However, research on RVFV is hampered and restricted since the pathogen can only be handled under biosafety level 3 (BSL3) conditions. Therefore, it would be desirable to have tools at hand which allow to study aspects of RVFV infection cycle and the host response under non-BSL3 conditions.

WO 2007/104979 discloses a method for producing virus like particles for RVFV in a baculovirus system. Overby et al., J.Virol. (2006),pp. 10428-10435 describe the generation and analysis of VLPs of Uukuniemi virus as a system for studying Bunyaviral packaging and budding.

The systems disclosed in WO 2007/104979 and Overby et al. is not suitable for VLP mediated gene expression. The system of Overby et al. requires transfection of target cells with the polymeric constructs L and N prior to VLP infection. The RVFV VLPs of the present invention are capable of autonomous gene expression (primary transcription) without the need for additional manipulations of the target cells. Furthermore, it has not been shown that said VLPs can induce an antibody response and that they are suitable as vaccine. Concerning vaccination the article published by Overby et al. is not relevant since Uukuniemi virus is not pathogenic for animals or humans, respectively.

The establishment of a RVFV reverse genetics system which recapitulates the first steps of the replication cycle in a modular manner is disclosed. A minireplicon system was developed to measure RVFV polymerase activity. Additional expression of viral glycoproteins allowed efficient and helper-free packaging of minireplicon RNPs into virus-like particles (VLPs) which were released into the medium. By incubating naive cells with VLPs, the first steps of RVFV infection are reconstituted. Importantly, the system allowed to demonstrate that the L polymerase complexed with incoming RNPs is highly active in primary transcription and, if both L and the nucleoprotein N are additionally provided *in trans*, replication as well. The suitability of our modular approach was further validated by testing whether the antiviral protein MxA inhibits the primary and/or the secondary transcription of RVFV. The cytoplasmic human MxA which is encoded by chromosome 21 has antiviral activity against several viruses, in particular against Bunyaviruses.

The present invention as further defined in the claims is a method for producing virus-like particles whereby a mammalian cell line, preferably a human cell line, is transfected with several independent vectors. The cell lines are preferably derived from human origin since the virus-like particles are preferably used as vaccine which provides immunity against an infection of Rift Valley Fever Virus. The host cell is higher eukaryotic (for example, all mammals, including but not limited to mouse and human) and may be used for expression of the desired coding sequences when appropriate control sequences which are compatible with the designated host are used.

In a particularly preferred embodiment of the present invention the yield of the VLPs is increased by inhibiting the antivirally active kinase PKR. The kinase PKR can be inhibited by adding a potent PKR inhibitor, in particular a dominant negative mutant of PKR, for example PKR delta E7.

Mammalian cell lines available as hosts for expression of cloned genes are known in the art and include many immortalized cell lines available from the American Type Culture Collection (ATCC), such as HEp-2, CHO cells, Vero cells, baby hamster kidney (BHK) cells and COS cells or BSR-T7/5 cells, to name a few. Suitable promoters are also known in the art and include viral promoters such as that from SV40, Rous sarcoma virus (RSV), adenovirus (ADV), bovine papilloma virus (BPV), and cytomegalovirus (CMV).

Mammalian cells may also require terminator sequences, poly A addition sequences, enhancer sequences which increase expression, or sequences which cause amplification of the gene. These sequences are known in the art.

Preferred cell lines are selected from the group consisting of cell lines having the well-known scientific designations HEK 293, 293T, Vero, HeLa, COS-1, A549, Huh-7, Huh-7.5, Hep2G, Jurkat and/or BSR-T 7/5.

The advantage of the present method is that the cell lines do not contain infections with Rift Valley Fever Virus or inactivated RVFV virus at any time during the production of VLPs. Therefore, there is no risk that by recombinational events viable and infectious dangerous viruses emerge. The genes L and N, respectively, and the viral M segment, are cloned into a vector which can transiently express the open reading frames of the L and N genes, and the viral M segment. It is not necessary that the complete L and/or N gene, and/or M segment from RVFV is contained within the vectors. It is sufficient if the sequence coding for L and N, and the viral M segment, are present in a form which is at least 90% of the gene the segment calculated over the whole length of the gene the segment. According to the present invention at least a substantial part of the gene coding for L and/or N and/or the M segment is present in the vector. The term "at least a substantial part of the gene the segment" means that at least 90% and more preferred at least 95% of the wild type gene the segment are present.

Furthermore the cell line is transfected with a vector which is designated in the present application as a minireplicon plasmid. The minireplicon plasmid contains a multicloning site flanked by the non-coding 5'- and 3'- ends of the L, M or S segment of Rift Valley Fever Virus. The virus like RNA encoded by the minireplicon plasmid is not self-replicating but requires the coexpression of N and L genes which lead to transcription and replication.

In a particularly preferred embodiment of the present invention the autonomous transcription by the VLPs is enhanced by coexpressing the viral N gene (which is a transcriptional enhancer) with a gene of interest, e.g. a reporter, on a single minireplicon. Coexpression is achieved by using a minireplicon resembling the viral S segment, which encodes two genes in a so-called ambisense manner.

The advantage of this minireplicon is that an artificial gene segment can be inserted into the virus-like particles. Moreover, by packaging a suitable foreign gene into the VLP with the help of the minireplicon system it is possible to enhance the immunological activity and to improve the efficacy of VLPs in the immunization of patients and/or animals.

The inserted gene may be a gene obtained from RVFV like for example a gene coding for the Gn and/or Gc protein or the nucleoprotein N or the polymerase L. Expression of the N gene has already been achieved and proved to strongly enhance the immunogenicity of the RVFV-VLPs (see example 7). Alternatively the gene may code for a protein which stimulates the immune response. It may for example code for a ligand to which a receptor of immune cells may bind or alternatively interferons like IFN-beta, immuncytokines like IL-2, CXCL-10. In the experiments of the present application the minireplicon contained a luciferase gene obtained from *Renilla reniformis* as model system in order to show that the gene contained within the minireplicon is expressed. In the vaccination experiments VLPs comprising an artificial gene segment preferably encoding the RVFV N gene fragment were used.

In a preferred embodiment the minireplicon is transcribed with the help of an RNA-polymerase I promoter which may be obtained from human origin and/or mouse. Alternatively the use of a T7 polymerase promoter is also possible.

The invention relates in another embodiment to virus-like particles (VLP) obtainable by the method of the present invention. Preferably such VLPs are used in pharmaceutical compositions as vaccine against an infection of RVFV. The VLPs of the present invention have several advantages. Since the VLPs are produced in mammalian, preferably human cell lines they have a glycosylation pattern on the protein which is specific for human. Therefore, there is no risk that undesired immunological reactions may occur. Such risk can, however, not be excluded when products are used which are produced in other organisms like plant cells and/or insect cells. The folding of the glycoproteins of the VLPs according to the present invention corresponds exactly to the usual folding of human proteins. Therefore, it can be concluded that immunological reactions of the immunized individual are specifically directed against proteins contained within the VLPs obtained from Rift Valley Fever Virus like N, L, G proteins.

In an alternative embodiment the virus-like particles are used for the vaccination of animals, in particular sheep and/or cattle. Since the virus can cause substantial losses in agriculture there is a demand to provide an efficient system for vaccination of animals like cows, sheep, horses, goats or camels. For preparing suitable VLPs it is preferred to use cell lines of the same species to be vaccinated. For the vaccination of cows, preferably bovine cell lines are used.

An important advantage of the VLPs of the present application is that their proteins correspond exactly to the proteins derived from the virus without, however, the risk that an attenuated virus used for immunization may be reactivated by undesired recombination events resulting in undesired side-effects.

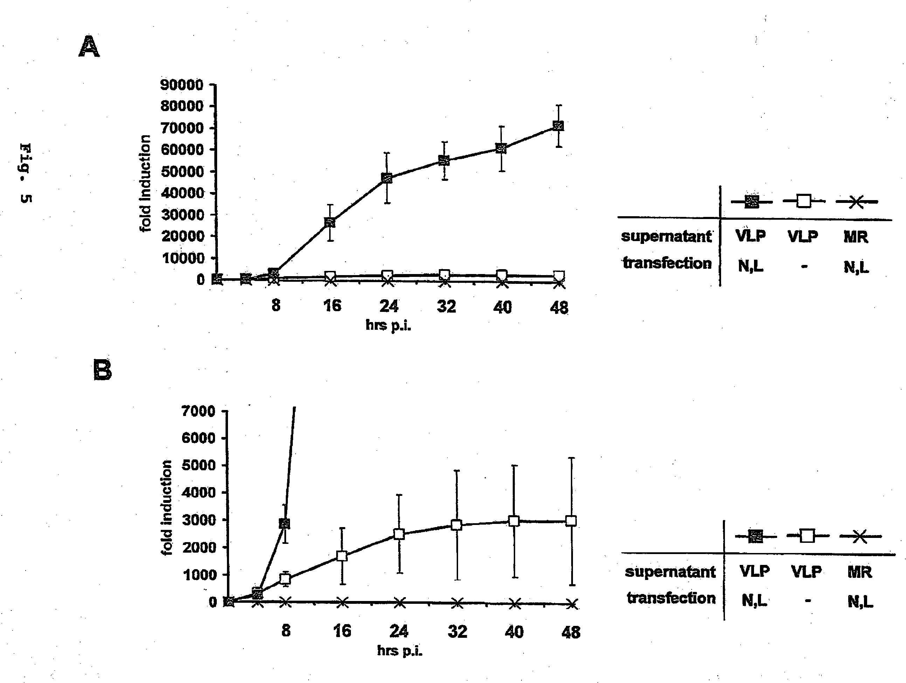

A further advantage of the present invention is the ability to autonomously transcribe any gene encoded on the VLP minireplicon RNA. This so-called primary transcription allows to express the gene of interest in infected cells without any further manipulation such as co-transfection of L and N gene (as shown for example in Fig. 5B).

The virus-like particles of the present invention can be used in a method for testing whether an agent has antiviral activity against Rift Valley Fever Virus. This test method is a kind of screening method wherein VLPs prepared according to the teaching of the present application are brought into contact with a mammalian cell line which can be infected by the VLPs. Additionally, an agent which is supposed to have antiviral activity is added to the test sample. In a comparative test the same amount of the VLPs and cells of the same cell line are brought together whereby the agent to be tested is replaced by a suitable control.

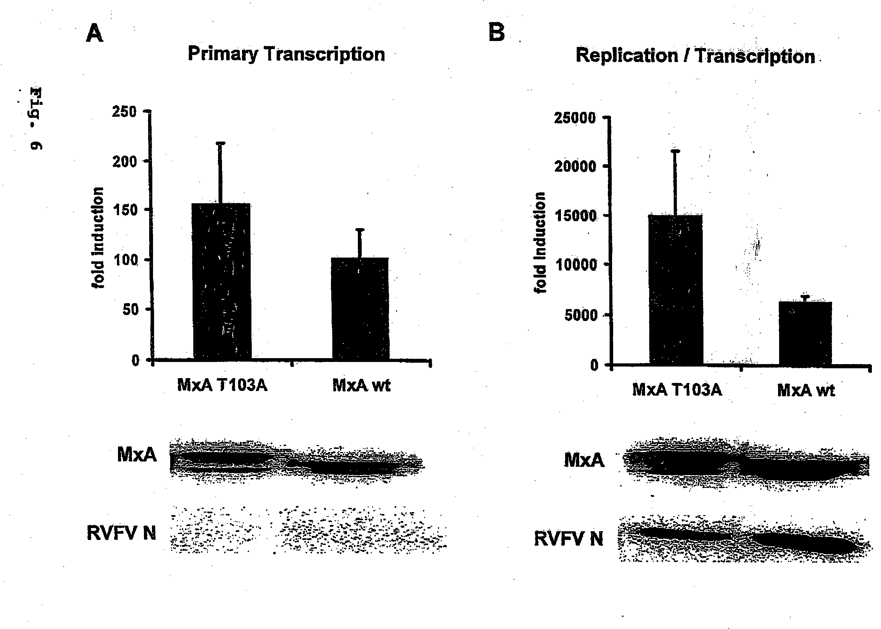

The control can be either the corresponding amount of buffer without any agent or it can be a corresponding compound which does not have an antiviral activity. One example of the agent to be tested is the antiviral human protein MxA. In the examples (see Figure 6) the human protein MxA has been tested in an active form compared with an inactive form.

Furthermore, the testing method is suitable for determining the activity of neutralizing antibodies having activity against RVFV or for testing any chemically synthesized compound or compounds derived from naturally occurring substances which are supposed to have antiviral activity. The advantage of the test method is that it can be performed under relatively low security conditions, since no viable virus is used. Furthermore, the test can be easily automated, standardized and a high throughput of compounds to be screened is possible.

A screening test for agents having antiviral activity can be performed in the following manner. A suitable cell line (e.g. BHK cells) were seeded in equal amounts into plates having 96 wells. After growth of the cells for one day, about 50 µl of a VLP solution containing about 10⁴ active VLPs are dispensed in each well and the cells are incubated for one hour. Thereafter to each well an aliquot of about 50 µl medium is added whereby the test solution contains the agent to be tested and the control does not contain the agent to be tested. After incubation over night the cells are suspended in 20 µl passive lysis buffer and the *Renilla* activity is measured. By using microtiter plates having for example 96 wells it is easy to perform the test in an automated manner by using suitable laboratory automated devices.

The present invention is shown in the enclosed Figures and preferred embodiments are described in the examples.

### Description of Figures

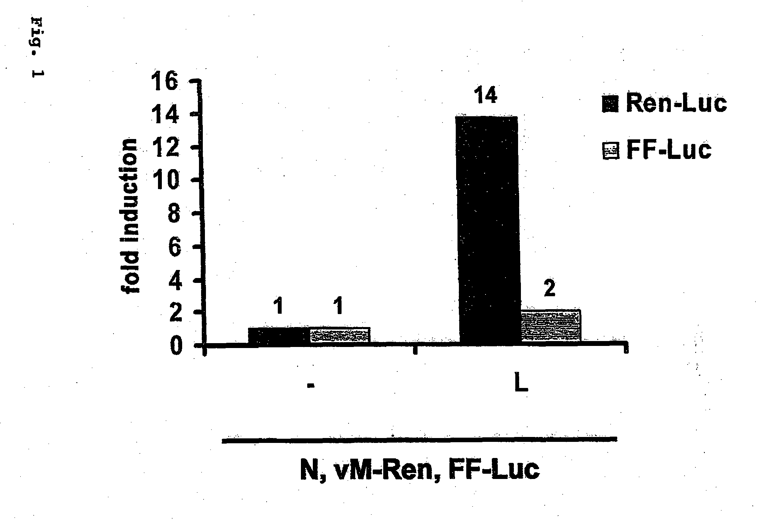

**Fig. 1****: Minireplicon system for RVFV.**
   Human 293T cells were transfected with expression plasmids for the nucleoprotein N and the viral polymerase L, together with a minireplicon construct containing the Ren-Luc reporter gene in antisense flanked by the untranslated regions of the M segment (vM-Ren). As a negative control the L expression plasmid was omitted. An FF-Luc plasmid was cotransfected to control for background expression. Cell lysates were assayed 24 h post transfection for Ren-Luc and FF-Luc activity. Luciferase counts were normalized to the experiment without L, numbers on top of each column indicating fold induction. Data from a representative experiment are shown.

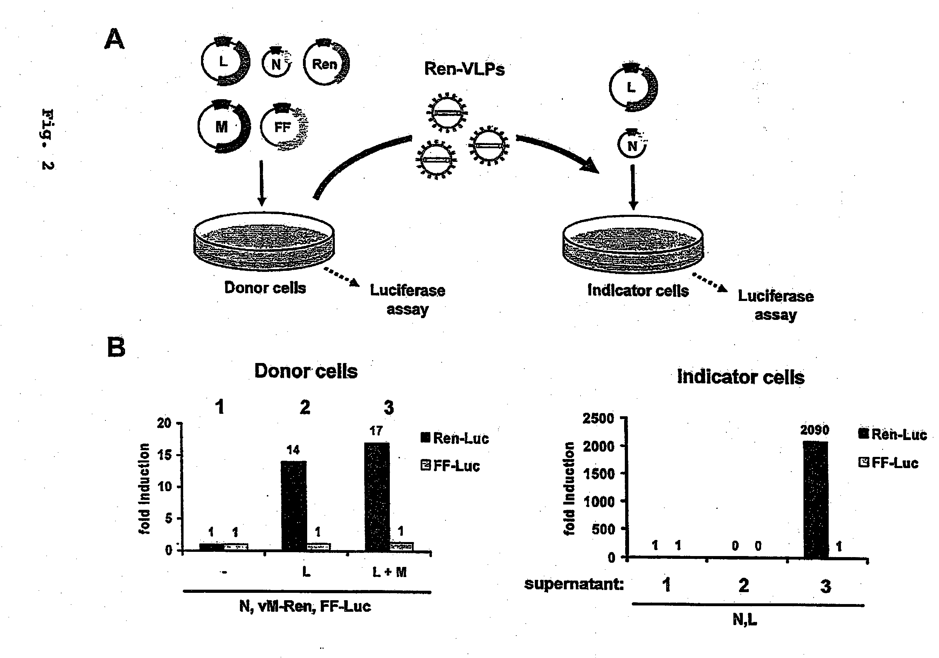

**Fig. 2****: Formation of RVFV VLPs.**
   (A) Outline of the procedure used for generation of VLPs. Donor cells were transfected with expression plasmids for N, L and the viral glycoproteins (M), together with the reporter minireplicon construct vM-Ren. Release of VLPs into the supernatant of donor cells was detected by transfer of supernatants on indicator cells expressing both N and L. (B) Luciferase activities in donor and indicator cells. 293T cells (donor cells) were transfected with minireplicon system plasmids alone as described in Fig. 1 (left panel, columns 1 and 2), or together with an M segment expression plasmid (left panel, column 3). Supernatants were harvested 24 h later and used to infect BSR-T7/5 indicator cells (right panel). FF-Luc and Ren-Luc activities in donor cells were measured upon removal of supernatants, and indicator cells were analyzed 24 h post infection. Luciferase counts were normalized to the experiment where L has been omitted, and numbers on top of each column display fold induction. Data from a representative experiment are shown.

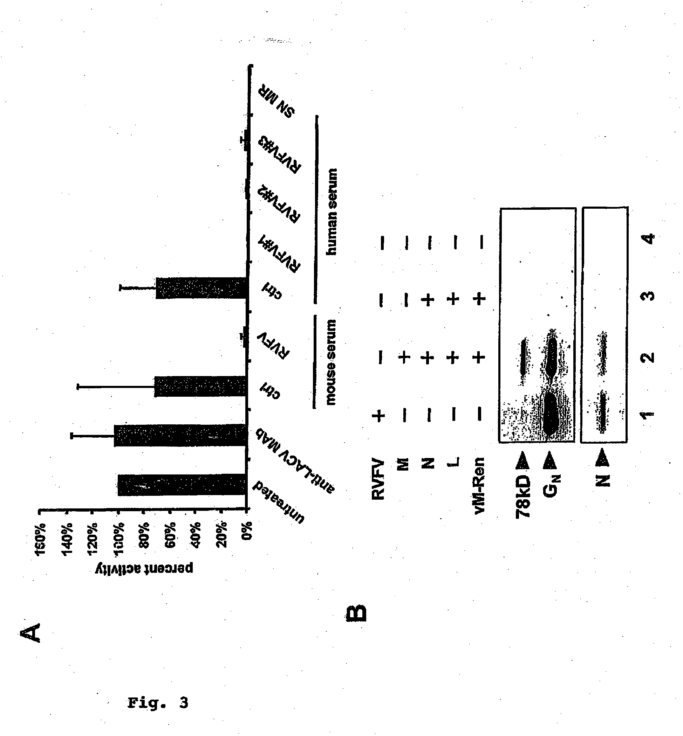

**Fig. 3****: VLPs resemble authentic RVFV particles.**
   (A) Neutralisation of VLPs by RVFV-specific antisera. VLP-containing supernatants were incubated with 5 µl of mouse or human antisera specific for RVFV for 1 hour at 37°C. As controls, 5 µl of a non-seroconverted mouse and human (ctrl) or of a monoclonal antibody against La Crosse bunyavirus glycoprotein Gc were used. Cells were then infected with antibody-treated or untreated VLP preparations, or the supernatant from minireplicon-expressing cells (SN MR). Ren-Luc activity was measured 24 hours later and is shown as percent activity of untreated VLPs. Error bars show standard deviations from three independent experiments. (B). Western blot analysis of RVF virus particles and VLPs. Concentrated supernatants from RVFV-infected cells (lane 1) and 293T cells transfected with different plasmid combinations (lane 2 to 4) were analyzed by Western blotting using a monoclonal antibody specific for RVFV Gn and 78kD protein (upper panel), and an antibody against the nucleoprotein N (lower panel).

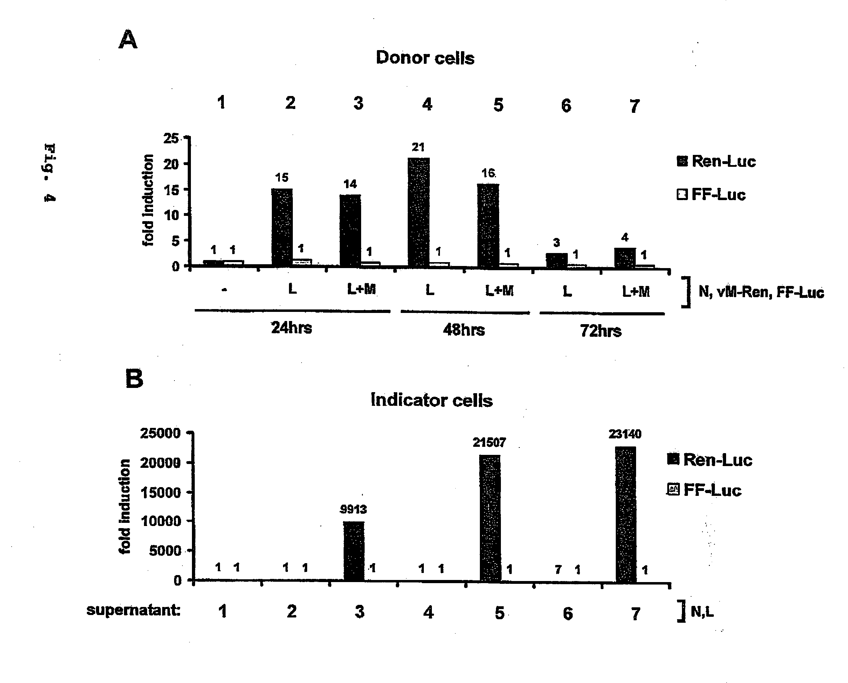

**Fig. 4****: Optimisation of VLP production.**
   VLPs were generated as described in Fig. 2, and VLP release from donor cells was followed over the course of three days. Supernatants from parallel dishes were harvested at the timepoints indicated and used to infect indicator cells expressing N and L. (A) Ren-Luc and FF-Luc activities in donor cells were measured upon removal of supernatant. (B) Analysis of luciferase activities in indicator cells was performed 24 h post-infection. Luciferase counts were normalised to the experiment where L has been omitted. Data from a representative experiment are shown, numbers on top of each column displaying fold induction.
**Fig. 5****: Primary and secondary transcription in VLP-infected cells.** (A). BSR-T7/5 cells were either mock transfected (open squares), or transfected with N and L expression plasmids (filled squares) prior to infection with VLPs harbouring Ren-Luc RNPs. As a negative control, cells transfected with N and L plasmids were infected with supernatants from minireplicon-expressing donor cells (crosses). Ren-Luc activities in VLP-infected cells were then measured at different timepoints over the course of 48 h. (B). The same data is shown as in A, but with the y-axis adjusted to visualise activity of VLPs in naive cells. Error bars show standard deviations from three independent experiments.
**Fig. 6****: Inhibition of both RVFV primary and secondary transcription by human MxA.**
   The influence of human MxA on RVFV primary transcription was analyzed by transfecting BSR-T7/5 cells with 1.5µg of an expression construct for wt MxA (A). To study the effect of MxA on RVFV replication and secondary transcription, the same amount of plasmid for wt MxA was cotransfected together with 0.5µg of each N and L (B). The inactive MxA-mutant T103A was used as a negative control. Twenty-four hours post transfection, cells were infected with VLPs harbouring Ren-Luc RNPs. *Renilla luciferase* activities in cell lysates were measured 24 hours later. Equal amounts of these lysates were used for Western Blot analysis to confirm expression of both MxA and RVFV N. Data are shown from three independent experiments performed in parallel, error bars indicating standard deviations.

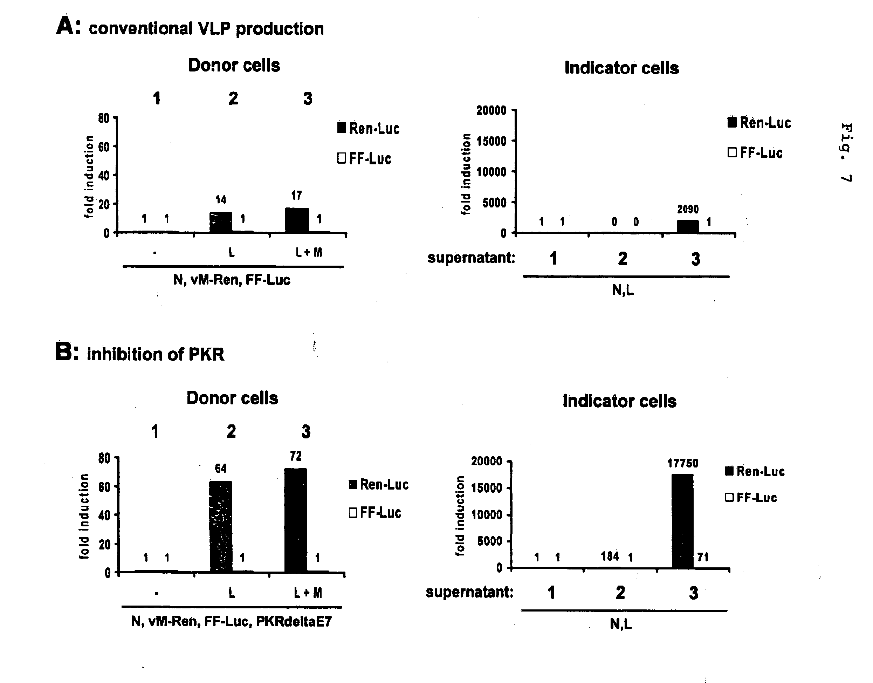

**Fig. 7****: Increasing yield of VLPs.**
   Fig. 7 demonstrates the increasing of VLP yields by co-expression of the PKR-Inhibitor PKRdeltaE7. Fig. 7 (A) Donor cells (left panels) were either conventionally transfected with VLP constructs (column 3), or (B) additionally transfected with 0.5 µg of the expression construct pl.18-PKRdeltaE7. As specificity control was the M-expression construct (coding for the glycoproteins) omitted (column 2), as negative control we omitted the polymerase L as well as M. (column 1). Supernatants of donor cells were harvested at 48 h post-transfection and cells were lysed for luciferase assays. Indicator cells were transfected with N- and L-expression constructs 16 h before infecting them with 250 µl VLP-containing donor cell supernatants. Luciferase activities of the indicator cells taken 24 h after VLP infection show that -as expected - only the full set of VLP constructs (column 3) leads to production of VLPs. Addition of pl.18-PKRdeltaE7 into the VLP-transfection mix results in a ca. 10-fold increase of VLP activity (compare column 3 of indicator cells in A and B).

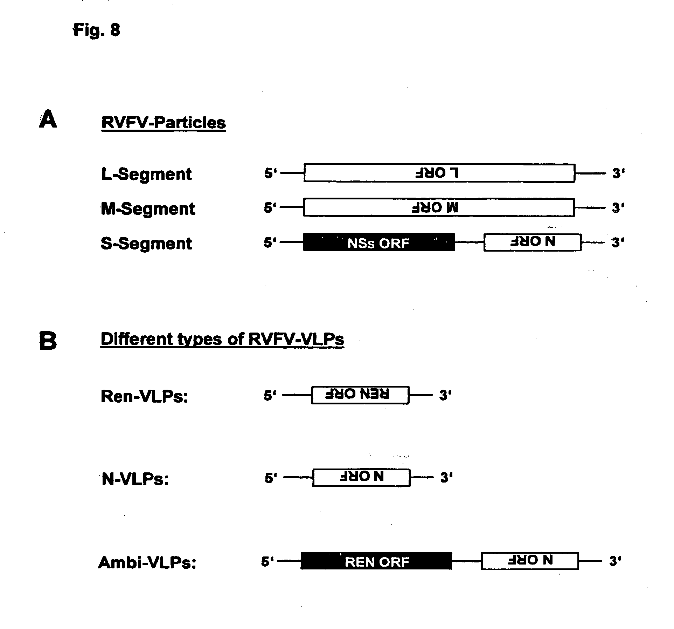

**Fig. 8****: Coding strategies.**
   Fig. 8 demonstrates the coding strategies of RVFV and RVFV-VLPs. Gene segments of RVFV (A) and genome segments of the different types of RVFV-VLPs (B). Note that Ren-VLPs and N-VLPs are based on the viral M segment, whereas the ambisense VLPs are based on the S segment. The Ren-ORF of the Ambi-VLPs can be replaced by any gene of interest.
**Fig. 9****: Enhancing autonomous VLP transcription by using ambisense-minireplicons**

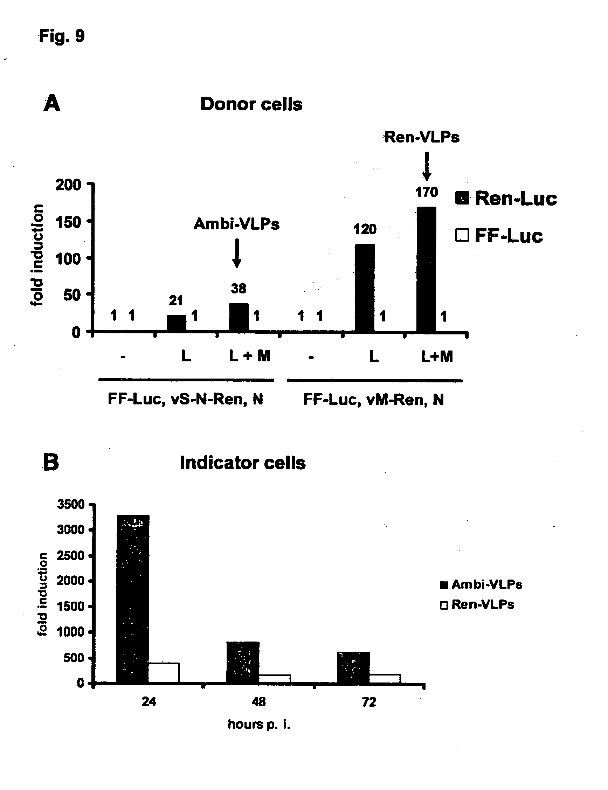

   Fig. 9 demonstrates the comparison of ambisense-VLPs with conventional (Ren-) VLPs. (A) For production of the VLPs, 293T cells (donor cells) were transfected in 6-well dishes with 0.5 µg pl.18-N, 0.5µg minireplicon construct (pHH21-vS-N-Ren for Ambi-VLPs and pHH21-vM-Ren for Ren-VLPs), 0.1µg pGL3 ctrl, 0.5µg pl.18-L, and 0.5 µg pl.18-M. 48 h later supernatants were harvested and donor cells lysed. Relative reporter activities of lysates are shown as columns. The negative control (omission of the L construct from the transfection mix) was set as 1. (B) Autonomous VLP activities. BHK indicator cells in 12-well dishes were incubated with 250 µl VLP supernatant (see A: L+M) lysed at the indicated time points, and the Ren-activities were measured.

### Example 1: Construction of Plasmids

All plasmids were generated using standard molecular cloning techniques and confirmed by DNA sequencing. PCR was carried out with AccuPrime Pfx DNA Polymerase (Invitrogen; for cloning purposes) or Taq DNA Polymerase (Eppendorf; for diagnostic purposes and addition of single adenosines for TA cloning). TA cloning was done using the pcDNA3.1/V5-His TOPO TA Expression Kit (Invitrogen) according to the manufacturers instructions. The preparation of RVFV first-strand cDNA was performed as described previously for La Crosse virus (Blakqori et al., J.Gen.Virol. 84 (2003), pp 1207-1214).

Viral genes were amplified from the first-strand cDNA using specific primers as shown in Table 1.

**Table 1a: DNA oligonucleotide primers**

| **Name** | **SEQ ID NO.** | **Sequence (5' to 3')^{a}** | **Segment/Gene** | **Nucleotides^{b,c}** |
|---|---|---|---|---|
| RVFV_SapI_L1for | 1 | GACAGAGCTCTT**CATCATGGATTCTATATTATCAAAACAGCTG** | L gene | 16-45 |
| RVFV_L1rev | 2 | **TGAGGCATCCATTGCTGC** | L gene | 1959-1942 |
| RVFV_L2for | 3 | **GCTGGGCCTTTGATCTCTC** | L gene | 1727-1745 |
| RVFV_L2rev | 4 | **CTCCCGATGACCATCCAG** | L gene | 3159-3142 |
| RVFV_L3for | 5 | **GAGGAAAGAATTGTTCAATCGG** | L gene | 2818-2839 |
| RVFV_L3rev | 6 | **AATGGTCACGGATAACCATAGC** | L gene | 4867-4846 |
| RVFV_L4for | 7 | **AAGCAACTCTAGCTCACACCCC** | L gene | 4712-4733 |
| RVFV_L4rev_SapI | 8 | GACAGAGC**TCTTCTGGTCTTAGCCTAGCATGTCATC** | L gene | 6302-6280 |
| RVFV_SapI_M1for | 9 | GACAGAGCTCTTC**TAAATGTATGTTTTATTAACAATTCTAATCTCG** | M gene | 18-50 |
| RVFV_M1rev | 10 | **TCAAACAAGCCTCTGCCC** | M gene | 2217-2200 |
| RVFV_M2for | 11 | **GTGAACAGGGAAATAGGATGG** | M gene | 1956-1976 |
| RVFV_M2rev_SapI | 12 | GACAGAGCTCTTCC**TGATCTATGAGGCCTTCTTAGTG** | M gene | 3619-3596 |

| | | | | |
|---|---|---|---|---|
| ^{a}nucleotides corresponding to RVFV sequences are in boldface letters ^{b}nucleotide numbers according to the database entries DQ375403 (L segment), DQ380206 (M segment), and DQ380151 (S segment) ^{c}hybridization sites for reverse primers are indicated by reverse order numbering | | | | |

**Table 1b: DNA oligonucleotide primers**

| **Name** | **SEQ ID NO.** | **Sequence (5' to 3')^{a}** | **Segment/ Gene** | **Nucleotides^{b,c}** |
|---|---|---|---|---|
| RVFV _SapI_Sfor | 13 | GACAGAGCTCTTC**ATAATGGACAACTATCAAGAGCTTGC** | N gene | 36-61 |
| RVFV_C13_Srev_SapI | 14 | GACAGAGCTCTTC**CAAGCAGCAAAAGAGGACATTTC** | N gene | 1062-1040 |
| RVFV_cMPro1for | 15 | GACAGACGTCTCCTATAG**ACACAAAG**ACGGTGCATTAAATGAAGAGCGGTACCGCTCTTC**ATCAGTACGTGTAAAAGCAA** | MUTR | 1-//-3634 |
| RVFV_cMPro2rev | 16 | **TGACCTATCTGTACAATACTTATACTTACATAATTAGGTTTGCTTATGTCTACTTATTTCAACATATTGCTTTTACACGTACTGAT** | MUTR | 3694-3615 |
| RVFV_cMPro3for | 17 | **AGTATTGTACAGATAGGTCAAATTATTGGAATATCCAAGCTTAGAAACTTATGCAATAATACTTTAGATGTAAGCTTAGT** | M UTR | 3675-3754 |
| RVFV_cMPro4rev | 18 | **ACTAGAATATTCACAAGCACTTGAGACTCCTGCTGCCTCACCCCACCACCCCAAATTACAACTAAGCTTACATCTAAAGT** | M UTR | 3814-3735 |
| RVFV_cMPro5for | 19 | **GTGCTTGTGAATATTCTAGTTGGCGTAATCGTCTTTTGCCAGATTAGCTGGGAATTAAACTAACTCTTTGAAGTTGCACC** | M UTR | 3795-3874 |
| RVFV_cMPro6rev | 20 | TCTGTCCGTCTCCACCC**ACACAAAGACCGGTGCAACTTCAAAGAGTTA** | MUTR | 3885-38555 |
| RVFV_vMPro_pHH21for | 21 | GACAGACGTCTCCTATT**ACACAAAGACCGGTGCAACTTC** | - | - |
| RVFV_vMPro_pHH21rev | 22 | GACAGACGTCTCCGGG**ACACAAAGACGGTGCATTAA** | - | - |
| RVFV_MRen_SapIfor | 23 | GACAGAGCTCTTCAAA*ATGACTTCGAAAGTTTATGATCCAG* | - | - |
| RVFV_MRen_SapIrev | 24 | GACAGAGCTCTTCTTGAC*TTATTGTTCATTTTTGAGAACTCGC* | - | - |

| | | | | |
|---|---|---|---|---|
| ^{a}nucleotides corresponding to RVFV sequences are in boldface letters ^{b}nucleotide numbers according to the database entries DQ375403 (L segment), DQ380206 (M segment), and DQ380151 (S segment) ^{c}hybridization sites for reverse primers are indicated by reverse order numbering | | | | |

The constructs pl.18_RVFV_L (SEQ ID NO:25), pl.18_RVFV_M (SEQ ID NO:26) and pl.18-RVFV_N (SEQ ID NO:27) contained the appropriate coding sequences subcloned into the eukaryotic high-level expression plasmid pl.18 (kindly provided by Jim Robertson, National Institute for Biological Standards and Control, Hertfordshire, UK). The minireplicon pHH21-vMRen (SEQ ID NO:28) was also constructed.

For construction of pl.18_RVFV_L, the L segment coding region was assembled in a stepwise manner from four overlapping cDNA fragments. These fragments were generated by PCR using primer pairs RVFV_Sapl_L1for/RVFV_L1rev, RVFV_L2for/RVFV_L2rev, RVFV_L3for/RVFV_L3rev, and RVFV_L4for/RVFV_L4rev_Sapl. The PCR fragments were individually TA-cloned into the pcDNA3.1-TOPO Vector (Invitrogen), giving rise to plasmids pcDNA3.1_RVFV_L1, pcDNA3.1_RVFV_L2, pcDNA3.1_RVFV_L3, and pcDNA3.1_RVFV_L4. Then, the insert of pcDNA3.1_RVFV_L2 was cut out with *Eco*RI and *Xho*I and cloned 3' of the L1 insert into the *Eco*RI/*Xho*I-digested pcDNA3.1_RVFV_L1. The resulting plasmid was named pcDNA3.1_RVFV_L1+L2.

In parallel, fragment L4 was subcloned into pl.18 using the *Bam*HI and Xhol restriction sites. The resulting plasmid, pl.18-L4 was then digested with Sacl and *Eco*RI. The gel-purified L4 fragment flanked by *Sac*I/*Eco*RI restriction sites at the 5' and 3' end, respectively, was then subcloned together with the *Bam*HI/*Sac*I-digested L3 fragment (cut out of pcDNA3.1_RVFV_L3) into the *Bam*HI/*Eco*RI-digested pl.18 vector. The resulting construct, pl.18_L3+L4, was then reopened with *Bam*HI and *Apa*I and the joined with the L1+L2 fragment cut out of pcDNA3.1_RVFV_L1+L2, again using *Bam*HI/*Apa*I. The amino acid sequence of the full-length insert in pl.18-RVFV L corresponds to the database entry DQ375403.

For construction of pl.18_RVFV_M two overlapping fragments were generated using primer pairs RVFV_Sapl_m1for/RVFV_m1rev and RVFV_m2for/RVFV_m2rev_Sapl. The two fragments were TA-cloned to generate plasmids pcDNA3.1_RVFV_M1 and pcDNA3.1_RVFV_M2, respectively. To obtain pl.18_RVFV_M, both fragments were finally cloned into the pl.18 vector in a three-way ligation reaction, where an internal *DraIII*-site served for joining of M1 and M2, and *KpnI* and *XhoI* restriction sites were used for ligation into pl.18. The coding sequence of the insert corresponds to the database entry DQ380206, with the difference of one amino-acid substitution from leucine to glutamine at position 232.

Plasmid pl.18_RVFV_N was constructed by amplification of first-strand cDNA using PCR primers RVFV_Sapl_Sfor and RVFV_C13_Srev_Sapl. Subcloning of the resulting fragment into the TOPO vector first gave rise to plasmid pcDNA3.1_RVFV_N, from which the fragment was then cloned into the pl.18 vector using *KpnI*/*XhoI* sites. The sequence of the insert corresponds to database entry DQ380151.

The reporter plasmid pHH21_RVFV_vMRen contains the *Renilla* luciferase gene (Ren-Luc) in antisense orientation, flanked by the 3' and 5' genomic untranslated regions (UTRs) of the RVFV M segment. This reporter plasmid was generated in a three-step manner. First, the M UTRs seperated by a linker sequence containing two flanking *SapI* restriction sites and one central *Kpnl* site were constructed from overlapping oligonucleotides RVFV_cMPro1for, RVFV_cMPro2rev, RVFV_cMPro3for, RVFV_cMPro4rev, RVFV_cMPro5for, RVFV_cMPro6rev as described previously (XXX). The resulting PCR fragment was TA-cloned to obtain pcDNA3.1_RVFV_MPro. Then, the UTR sequences including the linker were reamplified using primer pair RVFV_vMPro_pHH21for/ RVFV_vMPro_pHH21rev and cloned into the pHH21 backbone vector via primer-encoded *Esp3l* sites. This gave rise to precursor plasmid pHH21_RVFV_vMPro. In a last step, the Ren-Luc gene was amplified from plasmid pRL-SV40 (Promega) using primers RVFV_MRen_Saplfor and RVFV_MRen_Saplrev, and inserted into pHH21_RVFV_vMPro using *Sapl,* restriction sites, resulting in plasmid pHH21_RVFV_vMRen.

Plasmid pGL3-control (Promega) expresses firefly luciferase (FF-Luc) under control of a mammalian RNA polymerase II promoter.

Plasmid constructs encoding human MxA and the GTPase-deficient mutant T103A have been described previously [Ponten et al., J. of Virology 71:2591-9 (1997)]. For improved expression in mammalian cells we cloned the corresponding coding sequences into pl.18 via BamHI/EcoRI restriction sites, giving rise to plasmids pl.18-MxA and pl.18-HA-MxA(T103A).

### Example 2: RVFV Minireplicon system

Geneticin G418 (Biochrom AG) was dissolved in H₂O to 100mg/ml and used at a concentration of 1 mg/ml cell culture medium. BSR-T7/5 cells, Vero cells and 293T cells were cultivated in Dulbecco's modified Eagle's medium (DMEM) supplemented with 10% FCS.

Subconfluent monolayers of 293T cells in 12-well plates were transfected with 0.4 µg each pl.18_RVFV_N and pl.18_RVFV_L, 0.25µg pHH21_RVFV_vMRen, and 0.1µg pGL3-control using 3.5 µl Fugene HD transfection reagent (Roche) in 100 µl serum free medium (OptiMEM; Invitrogen). In some experiments, specific expression plasmids were omitted from the transfection mix. After transfection, cells were incubated for the indicated times and lysed in 100 µl Dual Luciferase Passive Lysis Buffer (Promega). An aliquot of 20 µl of the lysate was assayed for FF-Luc and Ren-Luc activities as described by the manufacturer (Promega).

Minireplicon systems are useful tools to investigate viral polymerase activity and promoter sequences. For bunyaviruses, they usually consist of a reporter gene flanked by viral 5' and 3' UTRs, and recombinant L and N proteins. The reporter construct is transfected into mammalian cells along with the L and N expression constructs and will be transcribed intracellularly into a minireplicon resembling a viral gene segment. The products of the expression constructs faithfully encapsidate, transcribe and replicate the minireplicon, thus resulting in reporter activity. For RVFV, minireplicon systems have been established which are based on the coexpressed RNA polymerase of the bacteriophage T7.

We disclose a T7-independent version in which the expression constructs were under control of a promoter for the cellular RNA polymerase II (RNAP II), and the minireplicon was transcribed intracellularly by RNAP I. The L and N genes were cloned into the high expression vector pl.18, and a minireplicon consisting of the antisense gene for the *Renilla* Luciferase (Ren-Luc) flanked by RVFV M segment UTRs was cloned into the RNAP I vector pHH21. Human 293T cells were transfected with these plasmids, and minireplicon activity was assessed after overnight incubation by measuring Ren-Luc activity of cell extracts.

Fig. 1 shows that coexpression of L, N and the minireplicon strongly upregulated the Ren-Luc activity (black bars), whereas omission of the L construct resulted in low activity. As the minireplicon plasmid was designed to produce negative sense RNA, a strong Ren-Luc activity clearly indicates successful packaging into RNPs and transcription by L and N. Moreover, the internal control consisting of the RNAP II-driven gene for the firefly luciferase (FF-Luc) was independent of the full set of RVFV plasmids (grey bars), as expected. Thus, our RVFV minireplicon system which is entirely based on the activity of cellular RNA polymerases was able to efficiently reconstitute recombinant RVFV RNPs.

### Example 3: Preparation of virus-like particles

Subconfluent monolayers of 293T cells in 6-well plates (donor cells) were transfected with 0.5µg of plasmids pl.18_RVFV_N, pl.18_RVFV_L, pl.18_RVFV_M, pHH21_RVFV_vMRen, and with 0.1µg of pGL3-control using 6.3µl Fugene HD transfection reagent. At the indicated timepoints supernatants were harvested and donor cells were lysed with 200µl Passive Lysis Buffer to measure luciferase activities as described above. The supernatants were frozen for at least one hour at -20°C, treated with 1 µl/ml Benzonase (Novagen) at 37°C for three hours, and centrifuged at 12.000 × g for 5 min to remove cellular debris. Aliquots of the treated supernatants were then used to infect infect new cells (indicator cells). In some experiments, indicator cells were transfected with L and N expression plasmids 16 h prior to the transfer of supernatant to support replication and transcription of RNPs.

For two bunyaviruses, Bunyamwera virus and Uukuniemi virus, VLP systems consisting of minireplicon RNPs packaged by viral glycoproteins were established and have been proven useful for the analysis of particle assembly and RNP packaging. To achieve this for RVFV, we cloned the reading frame of the viral M segment (encoding Gn, Gc, NSm, and the 78kD protein) into the RNAP II expression vector pl.18 (see Example 1). Immunofluorescense analyses using specific antisera confirmed expression of RVFV glycoproteins and their transport to the plasma membrane.

To generate VLPs, the M expression construct was cotransfected with the minireplicon system plasmids into 293T cells (donor cells). Twenty-four hours later, supernatants were harvested and used to infect BSR-T7/5 cells expressing RVFV L and N (indicator cells). An outline of the experiment is provided in Fig. 2A.

Two additional measures were taken to minimize unspecific reporter activities in indicator cells caused by carryover transfection. Firstly, any residual plasmids were destroyed by extensive treatment of VLP supernatants with DNase. Secondly, the species-specificity of the human RNAP I-driven minireplicon construct excluded background expression in the non-human indicator cells. Moreover, RNAP II-driven coexpression of FF-Luc in donor cells served as a specificity control, because the FF-Luc RNA lacks any RVFV sequences and should therefore not be packaged into VLPs.

Reporter assays shown in Fig 2B (left panel) demonstrate again the strong minireplicon activity mediated by L and N. Coexpression of the M segment-encoded glycoproteins had no detrimental effects.

Importantly, after transfer of supernatants to indicator cells, only the sample which had received the M expression construct in addition to the minireplicon system displayed a strong Ren-Luc activity. FF-Luc activity, by contrast, was neglectable in indicator cells. These data implicate that coexpression of the M segment-encoded genes results in efficient and specific packaging of minireplicon RNPs into VLPs, which are subsequently released into the medium, able to infect new cells.

We further analysed VLP characteristics by using neutralising antisera. VLP-containing supernatants were first incubated with different antisera derived from RVFV-infected mice or humans, and then used to infect indicator cells expressing RVFV L and N.

Proteins were separated by SDS-PAGE and transferred to a PVDF membrane (Amersham), followed by incubation in saturation buffer (PBS containing 10% non-fat dry milk and 0.05% Tween). The membrane was first incubated for one hour with primary antibodies, washed three times with 0.05% PBS-Tween, followed by incubation with a horseradish-peroxidase (HRP)-conjugated secondary antibody. After three additional washing steps, detection was performed using the SuperSignal West Femto chemiluminescence kit (Pierce).

Fig. 3A shows that a strong reduction in Ren-Luc activity occurs in receptor cells when RVFV-specific antisera were used. By contrast, a control antibody directed against the La Crosse virus envelope protein as well as sera from an uninfected mouse or individual had no neutralising activity.

The protein composition of VLPs was determined. To this aim, supernatants from 293T cells transfected with different plasmid combinations were purified and subjected to Western blot analysis (Fig. 3B). Supernatants from RVFV-infected cells served as positive control (lane 1). As expected, there was no release of viral proteins from minireplicon-expressing cells lacking glycoprotein expression (lane 3). In contrast, both the Gn and N protein could be detected in supernatants where the full set of plasmids for the VLP system had been transfected (lane 2), indicating that the composition of VLPs is similar to that of RVFV particles. We noted, however, that the relative proportion of the 78kD protein (which is recognized by the monoclonal antibody against Gn) was substantially higher in VLPs when compared to virus particles. Apparently, expression or particle incorporation of the M segment-ecoded proteins differs between plasmid-transfected and virus-infected cells. Together, these data indicate that the VLPs generated by coexpression of the minireplicon system and the M-encoded glycoproteins can serve not only as a model for authentic RVFV particles, but also for immunization of humans.

### Example 4 Concentration and optimization of RVF virus-like particles

### a) Concentration

Supernatants from VLP-expressing cells were collected and clarified from cell debris by centrifugation (12.000 x g, 10 min at 4°C). The particles were then concentrated by ultracentrifugation at 24,000 rpm and 4°C for 2 h in a Beckman SW41Ti rotor. The pellet was dried for 5 min before resuspension in phosphate-buffered saline (PBS).

Concentrated VLPs were used in the Western Blot shown in Fig. 3B and for vaccination.

### b) Optimization of VLP production

A time course analysis was performed to study the kinetics of VLP formation. Donor cells were transfected with either the minireplicon alone, or in addition with the M-expression construct to generate VLPs. From parallel dishes, cell lysates and supernatants were harvested after 24, 48 or 72 hours. Reporter assays of donor cell lysates showed again (compare Fig. 2B, left panel) that addition of the M segment expression plasmid had no substantial effect on minireplicon activity (Fig. 4A, columns 2 and 3). Both the minireplicon and the VLP donor cells displayed peak activity at 48 h post-transcription, and a decrease at the 72 h time point.

This indicates robust and continued production of recombinant RVFV components and, most probably, exhaustion after longer expression times. When supernatants of the time course were used to infect indicator cells, reporter assays confirmed again that the M segment expression plasmid was necessary to generate infectious VLPs (Fig. 4B). Moreover, VLP activities steadily increased in the supernatants harvested from 24 h to 72 h after transfection of donor cells. The apparent discrepancy between donor cells (peak at 48 h time point) and indicator cells (steady increase until 72 h) may simply be due to the fact that VLPs are relatively stable and accumulate in the supernatants over the whole time course, whereas gene expression in donor cells fades out.

Therefore, defined conditions for the highly efficient production of VLPs are disclosed.

### Example 5: Primary transcription of genes packaged in VLPs

Conventionally, primary transcription of negative-strand RNA viruses is measured by using translational inhibitors, e.g. cycloheximide. Cutting off the supply of newly synthesized viral proteins allows to measure the activity of incoming polymerase complexes without the amplification loop established by genome replication. However, the fact that bunyavirus mRNA transcription is strictly dependent on concurrent translation precludes a dissection of primary and secondary transcription by pharmaceutical means.

It was checked whether the VLP system would allow the measurement of RVFV primary transcription. Thus, BSR-T7/5 cells were infected with VLPs and their transcriptional activity was monitored by measuring Ren-Luc over a period of 48 h. To distinguish between primary and secondary transcription, the indicator cells were either left untransfected or were transfected with polymerase complex proteins N and L prior to infection (Fig. 5). In parallel, supernatants from minireplicon-expressing cells were used to detect potential background reporter activity. As expected, strong Ren-Luc activity was observed in VLP-infected cells where polymerase complexes were present (Fig. 5A). This activity steadily increased until 48 h post-infection, the last timepoint measured, thus being indicative for continuous replication and transcription. Supernatants from minireplicon-only transfected cells, by contrast, did not elicit any detectable Ren-Luc activity. Interestingly, a clear and reproducible Ren-Luc activity over background was also measured in VLP-infected naive (untransfected) cells, albeit to a much lower extent than in N and L-expressing cells. Maximum activity was reached after 24 h and remained at an almost constant level for further 24 h. This indicates that the VLP-associated viral polymerase is highly active in primary transcription. The same observation was also made in several other cell lines infected with VLPs. The established system thus allows, for the first time, to study primary transcription of a bunyavirus.

### Example 6: Effect of the antiviral protein MxA on VLP transcription

The VLP system of the present application offers the chance to dissect the effect of the antivirally active protein MxA on primary and secondary transcription of bunyaviruses. To investigate this, indicator cells were transfected with an expression construct for MxA and infected with RVFV VLPs. The MxA mutant T103A served as a negative control. Fig. 6A (upper part) shows that MxA expressed by indicator cells was capable of reducing the reporter activity of incoming VLPs, indicating an effect on primary transcription. Moreover, if N and L support constructs were expressed in addition to MxA, a similar, but somewhat stronger inhibitory effect was observed; although the VLP reporter activity was much higher as compared to the primary transcription set-up (Fig. 6B, upper part). Western blot analyses for MxA and RVFV N were performed to control recombinant gene expression (Fig. 6A and B, lower parts). These data suggest that MxA is capable of interfering with primary transcription of bunyaviruses and hence interacts not only with the soluble N protein, but also with assembled RNPs.

In summary, reverse genetics tools have been established which enable the study of RVFV RNP activity, virus assembly, virus entrance, primary transcription and antiviral mechanisms under non-BSL-3 conditions. Moreover, the modular buildup of those systems allows to dissect the viral life cycle in a systematic manner.

### Example 7: Vaccination of mice

VLPs according to the teaching of the present application whereby the VLPs contained the gene coding for N instead of the Renilla reporter gene (so-called N-VLPs) were produced and concentrated as described in Example 3 and 4a).

Two groups of six mice each (C57/BL6) were immunized intraperitoneally with either PBS (as control) or with 1,000,000 N-VLPs.

Two weeks after the immunization both groups of mice were challenged with 100,000 pfu (plaque-forming units) of the Rift Valley Fever Virus strain ZH548 intraperitoneally.

In the control group (PBS) five of six mice died.

From the group of mice immunized with N-VLPs according to the present invention all six mice survived the viral challenge.

It is very surprising that after a single vaccination a protection could be obtained after 14 days. Alternatively it is possible to repeat the immunization in such a manner that the animal is immunized two or three times after suitable periods of time ranging between two weeks and three months.

In the experiment a plasmid called pHH21_RVFV_vM-N was constructed containing the N-ORF of RVFV in a negative orientation flanked by the UTRs of the M segment. The N-ORF has been amplified by using appropriate primers.

### Example 8: Increasing VLP yields

The inhibition of the antivirally active kinase PKR increases VLP yields to a significant extent. PKR is activated by viral double-stranded RNA (dsRNA) or single-stranded RNA (ssRNA) containing a 5' triphosphate group and a short stem-loop structure (Sadler and Williams. Nat Rev Immunol 8:559-68 (2008)). PKR is a serine-threonine kinase that phosphorylates the alpha subunit of the eukaryotic translation initiation factor elF2, thereby leading to a translational arrest of cellular and viral mRNA translation. Some cells express a natural dominant-negative mutant of PKR called PKRDeltaE7 (Li, S. and A. E. Koromilas. J Biol Chem 276:13881-90 (2001). The expression of the VLP constructs as well as the activity of VLPs could be affected by PKR, since transfection and virus replication are known to activate PKR. Therefore, PKRDeltaE7 was used and this potent PKR inhibitor was coexpressed with the constructs for VLP production. The expression construct for PKRDeltaE7 was made by amplifying the cDNA Sequence by RT-PCR and cloning into the high-level expression vector pl.18 (which is also used for the VLP constructs). The experiment was performed as follows:
a) Subconfluent monolayers of 293T cells seeded in 6-well plates (donor cells) were transfected with 0.5 µg of plasmids pl.18_RVFV_N, pl.18_RVFV_L, pl.18_RVFV_M, pHH21_RVFV_vMRen, and with 0.1 µg of pGL3-control using Fugene HD transfection reagent. If required, 0.5 µg of pl.18_PKRDeltaE7 were added to the transfection mix. At 48 h post-transfection, supernatants were harvested and donor cells were lysed with 200 µl Passive Lysis Buffer to measure luciferase activities as described above. The supernatants were treated with 1 µl/ml Benzonase (Novagen) at 37°C for 3 h and centrifuged at 12,000 × g for 5 min to remove residual plasmids and cellular debris. Aliquots of the treated supernatants were then used to infect BSR-T7/5 cells (indicator cells). Replication and transcription of VLPs was supported by transfecting indicator cells with N and L expression plasmids pl.18_RVFV_N and pl.18_RVFV_L (0.5 µg each) 16 h prior to the transfer of supernatant.
b) The results in Fig. 7 show that co-transfection of 0.5 µg pl.18_PKRDeltaE7 in donor cells increases reporter activity in donor cells by a factor of about 5 (compare reporter activity of columns 2 and 3 in A vs. B). Importantly, co-transfection of pl.18_PKRDeltaE7 increased VLP production by a factor of about 10 (indicator cells, column 3, A vs. B). These results clearly indicate that PKR is inhibitory to VLP production, and that yields can be dramatically increased by blocking PKR.

Further experiments have shown that PKRDeltaE7 enhances the performance of reverse genetics systems of bunyaviruses in general.

### Example 9: Increase of the autonomous VLP transcription and immunogenicity

The viral genomic S segment of RVFV codes for two different viral genes (N and NSs) by a so-called *ambisense*-strategy. The N is transcribed like the other RVFV genes in minus-strand orientation, i.e. in a conventional manner. The NSs gene, by contrast, is transcribed from the other direction (Fig. 8 A). We exploited this dual expression strategy to produce VLPs which express the N together with another gene of interest (replacing NSs). Fig. 8 B shows the expression strategy of the VLPs described up to here, which express a single gene of interest (Ren-Luc or N), compared with the ambisense (Ambi-)VLPs.

The co-expressed N Gen of the ambisense VLPs is useful for two different purposes. On one hand increases N (which is part of the viral polymerase complex) the autonomous VLP expression (see below), on the other hand increases N expression the immunogenicity of the VLPs. The N protein of the RVFV-related Toscana virus is known to act as CTL antigen and may even induce neutralizing antibodies (Savellini et al., Virology 375:521-8 (2008)).

A comparison of the Renilla-activity of conventional Ren-VLPs with Ambi-VLPs demonstrates the enhancing effect of N expression (Fig. 9). The Ambi-VLP construct (vS-N-Ren) has in donor cells a lower basic activity as the M segment-based Ren-VLPs (Fig. 9 A). The reason for this is that the S segment promoter used for making the Ambi-VLPs has a lower basic activity as compared to the M promoter used for generating the Ren-VLPs (Gauliard et al., Virology 351:170-9 (2006)). Nonetheless show the Ambi-VLPs an approximately 5-fold stronger activity in indicator cells as Ren-VLPs, and even 3 days after VLPs infection there is still detectable activity (Fig. 9 B). Thus, the co-expression of the N gene on the Ambi-VLPs leads to a higher and more persevering VLP activity. To our knowledge, such an ambisense-VLP-System was not described so far.

Potential applications of the Ambi-VLP-System are: Marker vaccine (e.g. Renilla as Marker to dinstinguish infected from vaccinated animals), overexpression of immunity-enhancing cytokines, expression of small interfering RNAs, Expression of other viral proteins.

## Claims

1. A method for producing virus-like particles comprising
a) transfecting a mammalian cell line with several independent vectors comprising
aa) a vector containing at least 90% of the wild type segment of the M segment from Rift Valley Fever Virus,
bb) a vector containing at least 90% of the wild type gene of the L gene from Rift Valley Fever Virus,
cc) a vector containing at least 90% of the wild type gene of the N gene from Rift Valley Fever Virus and
dd) a vector containing a multicloning site flanked by the non-coding 5'-and 3'-ends of the L-, M- or S-segment of Rift Valley Fever Virus, and
b) culturing the transfected mammalian cell line under suitable conditions and obtaining the VLPs which are capable of autonomous gene expression from the supernatant of the transfected cultured cell lines.

2. Method according to claim 1 wherein the mammalian cell line is a human cell line.

3. Method according to claim 1 wherein the vector containing the non-coding 5'- and 3'-ends of the L-, M- or S-segment of the Rift Valley Fever Virus and a multicloning site contains an additional gene inserted within the multicloning site.

4. Method according to claim 3 wherein the gene coding for the luciferase obtained from *Renilla* is inserted within the multicloning site.

5. Method according to claim 3 wherein the gene coding for the N protein of Rift Valley Fever Virus has been inserted within the multicloning site.

6. Method according to claim 3 wherein the gene coding for the L protein of Rift Valley Fever Virus has been inserted within the multicloning site.

7. Virus-like particle comprising at least one protein of the Rift Valley Fever Virus obtainable by a method according to any of claims 1 to 6.

8. Pharmaceutical composition comprising a virus-like particle according to claim 7.

9. Use of virus-like particle according to claim 7 for the preparation of a vaccine of humans against an infection of Rift Valley Fever Virus.

10. Use of virus-like particle according to claim 7 for the preparation of a vaccine of animals against an infection of Rift Valley Fever Virus.

11. Method of testing an agent for antiviral activity against Rift Valley Fever Virus wherein
a) virus-like particles according to claim 7 are brought into contact with a mammalian cell line and the agent to be tested for antiviral activity and
b) the antiviral activity is measured by comparison with a control wherein the agent to be tested is replaced by a suitable control which does not have antiviral activity

12. Method according to claim 11 wherein the agent to be tested is selected from the group comprising anti-RVFV antibodies or fragments thereof, chemically synthesized compounds supposed to have antiviral activity or proteins, peptides, steroids of natural origin and derivatives thereof supposed to have antiviral activity against Rift Valley Fever Virus.

13. Testkit for performing a method according to claims 11 or 12, **characterized in that** the testkit comprises a mammalian cell line and virus-like particles according to claim 7.

## Patentansprüche

1. Verfahren zur Herstellung virusartiger Partikel, umfassend
a) das Transfizieren einer Säugerzelllinie mit mehreren unabhängigen Vektoren, umfassend
aa) einen Vektor, enthaltend mindestens 90 % des Wildtypsegments des M-Segments aus dem Rift-Valley-Fieber-Virus,
bb) einen Vektor, enthaltend mindestens 90 % des Wildtypgens des L-Gens aus dem Rift-Valley-Fieber-Virus,
cc) einen Vektor, enthaltend mindestens 90 % des Wildtypgens des N-Gens aus dem Rift-Valley-Fieber-Virus, und
dd) einen Vektor, enthaltend eine Mehrfachklonierungsstelle, flankiert von den nichtcodierenden 5'- und 3'-Enden des L-, M- oder S-Segments des Rift-Valley-Fieber-Virus, und
b) das Kultivieren der transfizierten Säugerzelllinie unter geeigneten Bedingungen und den Erhalt der VLPs, die zur autonomen Genexpression aus dem Überstand der transfizierten kultivierten Zelllinien fähig sind.

2. Verfahren nach Anspruch 1, wobei die Säugerzelllinie eine humane Zelllinie ist.

3. Verfahren nach Anspruch 1, wobei der Vektor, der die nichtcodierenden 5'- und 3'-Enden des L-, M- oder S-Segments des Rift-Valley-Fieber-Virus und eine Mehrfachklonierungsstelle enthält, ein zusätzliches, in die Mehrfachklonierungsstelle eingefügtes Gen enthält.

4. Verfahren nach Anspruch 3, wobei das für die aus *Renilla* erhaltene Luciferase codierende Gen in die Mehrfachklonierungsstelle eingefügt wird.

5. Verfahren nach Anspruch 3, wobei das für das N-Protein des Rift-Valley-Fieber-Virus codierende Gen in die Mehrfachklonierungsstelle eingefügt worden ist.

6. Verfahren nach Anspruch 3, wobei das für das L-Protein des Rift-Valley-Fieber-Virus codierende Gen in die Mehrfachklonierungsstelle eingefügt worden ist.

7. Virusartiges Partikel, umfassend mindestens ein Protein des Rift-Valley-Fieber-Virus, erhältlich durch ein Verfahren nach einem der Ansprüche 1 bis 6.

8. Pharmazeutische Zusammensetzung, umfassend ein virusartiges Partikel nach Anspruch 7.

9. Verwendung eines virusartigen Partikels nach Anspruch 7 zur Herstellung eines Impfstoffes für Menschen gegen eine Infektion mit dem Rift-Valley-Fieber-Virus.

10. Verwendung eines virusartigen Partikels nach Anspruch 7 zur Herstellung eines Impfstoffes für Tiere gegen eine Infektion mit dem Rift-Valley-Fieber-Virus.

11. Verfahren zum Testen eines Mittels auf antivirale Aktivität gegen das Rift-Valley-Fieber-Virus, wobei
a) virusartige Partikel nach Anspruch 7 mit einer Säugerzelllinie und dem auf antivirale Aktivität zu testenden Mittel in Kontakt gebracht werden und
b) die antivirale Aktivität durch Vergleich mit einer Kontrolle gemessen wird, wobei das zu testende Mittel durch eine geeignete Kontrolle ausgetauscht wird, die keine antivirale Aktivität aufweist.

12. Verfahren nach Anspruch 11, wobei das zu testende Mittel ausgewählt ist aus der Gruppe, umfassend Anti-RVFV-Antikörper oder Fragmente davon, chemisch synthetisierte Verbindungen, die antivirale Aktivität aufweisen sollen, oder Proteine, Peptide, Steroide natürlichen Ursprungs und Derivate davon, die antivirale Aktivität gegen das Rift-Valley-Fieber-Virus aufweisen sollen.

13. Testkit zum Ausführen eines Verfahrens nach den Ansprüchen 11 oder 12, **dadurch gekennzeichnet, dass** das Testkit eine Säugerzelllinie und virusartige Partikel nach Anspruch 7 umfasst.

## Revendications

1. Procédé de production de particules pseudo-virales, comprenant
a) la transfection d'une lignée de cellules de mammifère avec plusieurs vecteurs indépendants comprenant
aa) un vecteur contenant au moins 90 % du segment de type sauvage du segment M du virus de la fièvre de la vallée du Rift,
bb) un vecteur contenant au moins 90 % du gène de type sauvage du gène L du virus de la fièvre de la vallée du Rift,
cc) un vecteur contenant au moins 90 % du gène de type sauvage du gène N du virus de la fièvre de la vallée du Rift et
dd) un vecteur contenant un site de multiclonage flanqué par les extrémités 5' et 3' non codantes du segment L, M ou S du virus de la fièvre de la vallée du Rift, et
b) la culture de la lignée de cellules de mammifère transfectée dans des conditions appropriées et l'obtention des VLP qui sont capables d'une expression génétique autonome à partir du surnageant des lignées de cellules transfectées et mises en culture.

2. Procédé selon la revendication 1, dans lequel la lignée de cellules de mammifère est une lignée de cellules humaines.

3. Procédé selon la revendication 1, dans lequel le vecteur contenant les extrémités 5' et 3' non codantes du segment L, M ou S du virus de la fièvre de la vallée du Rift et un site de multiclonage contient un gène supplémentaire inséré au sein du site de multiclonage.

4. Procédé selon la revendication 3, dans lequel le gène codant la luciférase obtenue à partir de *Renilla* est inséré au sein du site de multiclonage.

5. Procédé selon la revendication 3, dans lequel le gène codant la protéine N du virus de la fièvre de la vallée du Rift a été inséré au sein du site de multiclonage.

6. Procédé selon la revendication 3, dans lequel le gène codant la protéine L du virus de la fièvre de la vallée du Rift a été inséré au sein du site de multiclonage.

7. Particule pseudo-virale comprenant au moins une protéine du virus de la fièvre de la vallée du Rift pouvant être obtenue par un procédé selon l'une quelconque des revendications 1 à 6.

8. Composition pharmaceutique comprenant une particule pseudo-virale selon la revendication 7.

9. Utilisation d'une particule pseudo-virale selon la revendication 7, pour la préparation d'un vaccin d'humains contre une infection par le virus de la fièvre de la vallée du Rift.

10. Utilisation de la particule pseudo-virale selon la revendication 7, pour la préparation d'un vaccin d'animaux contre une infection par le virus de la fièvre de la vallée du Rift.

11. Procédé de test d'un agent pour déterminer son activité antivirale contre le virus de la fièvre de la vallée du Rift, dans lequel
a) des particules pseudo-virales selon la revendication 7 sont mises en contact avec une lignée de cellules de mammifère et l'agent à tester pour son activité antivirale, et
b) l'activité antivirale est mesurée par comparaison avec un témoin, dans lequel l'agent à tester est remplacé par un témoin approprié qui n'a pas d'activité antivirale.

12. Procédé selon la revendication 11, dans lequel l'agent à tester est choisi dans le groupe comprenant les anticorps anti-RVFV ou leurs fragments, les composés synthétisés par voie chimique supposés avoir une activité antivirale ou les protéines, les peptides, les stéroïdes d'origine naturelle et leurs dérivées supposés avoir une activité antivirale contre le virus de la fièvre de la vallée du Rift.

13. Kit de test pour mettre en oeuvre un procédé selon la revendication 11 ou 12, **caractérisé en ce que** le kit de test comprend une lignée de cellules de mammifère et des particules pseudo-virales selon la revendication 7.


### SEQUENCE LISTING

<110> UniversitatsKlinikum Freiburg
<120> Rift Valley Fever Virus - like Particles and its use for immunisation
<130> ZEE20071108
<140> EP 08010548.9
   <141> 2008-06-10
<160> 28
<170> PatentIn version 3.5
<210> 1
   <211> 43
   <212> DNA
   <213> artificial sequence
<220>
   <223> primer
<400> 1
   gacagagctc ttcatcatgg attctatatt atcaaaacag ctg 43
<210> 2
   <211> 18
   <212> DNA
   <213> artificial sequence
<220>
   <223> primer
<400> 2
   tgaggcatcc attgctgc 18
<210> 3
   <211> 19
   <212> DNA
   <213> artificial sequence
<220>
   <223> primer
<400> 3
   gctgggcctt tgatctctc 19
<210> 4
   <211> 18
   <212> DNA
   <213> artificial sequence
<220>
   <223> primer
<400> 4
   ctcccgatga ccatccag 18
<210> 5
   <211> 22
   <212> DNA
   <213> artificial sequence
<220>
   <223> primer
<400> 5
   gaggaaagaa ttgttcaatc gg 22
<210> 6
   <211> 22
   <212> DNA
   <213> artificial sequence
<220>
   <223> primer
<400> 6
   aatggtcacg gataaccata gc 22
<210> 7
   <211> 22
   <212> DNA
   <213> artificial sequence
<220>
   <223> primer
<400> 7
   aagcaactct agctcacacc cc 22
<210> 8
   <211> 36
   <212> DNA
   <213> artificial sequence
<220>
   <223> primer
<400> 8
   gacagagctc ttctggtctt agcctagcat gtcatc 36
<210> 9
   <211> 46
   <212> DNA
   <213> artificial sequence
<220>
   <223> primer
<400> 9
   gacagagctc ttctaaatgt atgttttatt aacaattcta atctcg 46
<210> 10
   <211> 18
   <212> DNA
   <213> artificial sequence
<220>
   <223> primer
<400> 10
   tcaaacaagc ctctgccc 18
<210> 11
   <211> 21
   <212> DNA
   <213> artificial sequence
<220>
   <223> primer
<400> 11
   gtgaacaggg aaataggatg g 21
<210> 12
   <211> 37
   <212> DNA
   <213> artificial sequence
<220>
   <223> primer
<400> 12
   gacagagctc ttcctgatct atgaggcctt cttagtg 37
<210> 13
   <211> 39
   <212> DNA
   <213> Artificial Sequence
<220>
   <223> primer
<400> 13
   gacagagctc ttcataatgg acaactatca agagcttgc 39
<210> 14
   <211> 36
   <212> DNA
   <213> artificial sequence
<220>
   <223> primer
<400> 14
   gacagagctc ttccaagcag caaaagagga catttc 36
<210> 15
   <211> 80
   <212> DNA
   <213> artificial sequence
<220>
   <223> primer
<400> 15
   gacagacgtc tcctatagac acaaagacgg tgcattaaat gaagagcggt accgctcttc 60
   atcagtacgt gtaaaagcaa 80
<210> 16
   <211> 80
   <212> DNA
   <213> artificial sequence
<220>
   <223> primer
<400> 16
   tgacctatct gtacaatact tacataatta ggtttgctta tgtctactta tttcaacata 60
   ttgcttttac acgtactgat 80
<210> 17
   <211> 80
   <212> DNA
   <213> artificial sequence
<220>
   <223> primer
<400> 17
   agtattgtac agataggtca aattattgga atatccaagc ttagaaactt atgcaataat 60
   actttagatg taagcttagt 80
<210> 18
   <211> 80
   <212> DNA
   <213> artificial sequence
<220>
   <223> primer
<400> 18
   actagaatat tcacaagcac ttgagactgc tgctgcctca ccccaccacc ccaaattaca 60
   actaagctta catctaaagt 80
<210> 19
   <211> 80
   <212> DNA
   <213> artificial sequence
<220>
   <223> primer
<400> 19
   gtgcttgtga atattctagt tggcgtaatc gtcttttgcc agattagctg ggaattaaac 60
   taactctttg aagttgcacc 80
<210> 20
   <211> 48
   <212> DNA
   <213> artificial sequence
<220>
   <223> primer
<400> 20
   tctgtccgtc tccacccaca caaagaccgg tgcaacttca aagagtta 48
<210> 21
   <211> 39
   <212> DNA
   <213> artificial sequence
<220>
   <223> primer
<400> 21
   gacagacgtc tcctattaca caaagaccgg tgcaacttc 39
<210> 22
   <211> 36
   <212> DNA
   <213> artificial sequence
<220>
   <223> primer
<400> 22
   gacagacgtc tccgggacac aaagacggtg cattaa 36
<210> 23
   <211> 41
   <212> DNA
   <213> artificial sequence
<220>
   <223> primer
<400> 23
   gacagagctc ttcaaaatga cttcgaaagt ttatgatcca g 41
<210> 24
   <211> 43
   <212> DNA
   <213> artificial sequence
<220>
   <223> primer
<400> 24
   gacagagctc ttcttgactt attgttcatt tttgagaact cgc 43
<210> 25
   <211> 10608
   <212> DNA
   <213> Artificial Sequence
<220>
   <223> expression plasmid
<400> 25
   gtggcctaac tacggctaca ctagaagaac agtatttggt atctgcgctc tgctgaagcc      60
   agttaccttc ggaaaaagag ttggtagctc ttgatccggc aaacaaacca ccgctggtag     120
   cggtggtttt tttgtttgca agcagcagat tacgcgcaga aaaaaaggat ctcaagaaga     180
   tcctttgatc ttttctacgg ggtctgacgc tcagtggaac gaaaactcac gttaagggat     240
   tttggtcatg agattatcaa aaaggatctt cacctagatc cttttaaatt aaaaatgaag     300
   ttttaaatca atctaaagta tatatgagta aacttggtct gacagttacc aatgcttaat     360
   cagtgaggca cctatctcag cgatctgtct atttcgttca tccatagttg cctgactccc     420
   cgtcgtgtag ataactacga tacgggaggg cttaccatct ggccccagtg ctgcaatgat     480
   accgcgagac ccacgctcac cggctccaga tttatcagca ataaaccagc cagccggaag     540
   ggccgagcgc agaagtggtc ctgcaacttt atccgcctcc atccagtcta ttaattgttg     600
   ccgggaagct agagtaagta gttcgccagt taatagtttg cgcaacgttg ttgccattgc     660
   tacaggcatc gtggtgtcac gctcgtcgtt tggtatggct tcattcagct ccggttccca     720
   acgatcaagg cgagttacat gatcccccat gttgtgcaaa aaagcggtta gctccttcgg     780
   tcctccgatc gttgtcagaa gtaagttggc cgcagtgtta tcactcatgg ttatggcagc     840
   actgcataat tctcttactg tcatgccatc cgtaagatgc ttttctgtga ctggtgagta     900
   ctcaaccaag tcattctgag aatagtgtat gcggcgaccg agttgctctt gcccggcgtc     960
   aatacgggat aataccgcgc cacatagcag aactttaaaa gtgctcatca ttggaaaacg    1020
   ttcttcgggg cgaaaactct caaggatctt accgctgttg agatccagtt cgatgtaacc    1080
   cactcgtgca cccaactgat cttcagcatc ttttactttc accagcgttt ctgggtgagc    1140
   aaaaacagga aggcaaaatg ccgcaaaaaa gggaataagg gcgacacgga aatgttgaat    1200
   actcatactc ttcctttttc aatattattg aagcatttat cagggttatt gtctcatgag    1260
   cggatacata tttgaatgta tttagaaaaa taaacaaata ggggttccgc gcacttttcc    1320
   ccgaaaagtg ccacctgacg tctaagaaac cattattatc atgacattaa cctataaaaa 1380
   taggcgtatc acgaggccct ttcgtctcgc gcgtttcggt gatgacggtg aaaacctctg 1440
   acacatgcag ctcccggaga cggtcacagc ttgtctgtaa gcggatgccg ggagcagaca 1500
   agcccgtcag ggcgcgtcag cgggtgttgg cgggtgtcgg ggctggctta actatgcggc 1560
   atcagagcag attgtactga gagtgcacca tatgcggtgt gaaataccgc acagatgcgt 1620
   aaggagaaaa taccgcatca ggcgccattc gccattcagg ctgcgcaact gttgggaagg 1680
   gcgatcggtg cgggcctctt cgctattacg ccagctggcg aaagggggat gtgctgcaag 1740
   gcgattaagt tgggtaacgc cagggttttc ccagtcacga cgttgtaaaa cgacggccag 1800
   tgccaagctt gcatgcctgc aggtcaattc cctggcatta tgcccagtac atgaccttat 1860
   gggactttcc tacttggcag tacatctacg tattagtcat cgctattacc atggtgatgc 1920
   ggttttggca gtacatcaat gggcgtggat agcggtttga ctcacgggga tttccaagtc 1980
   tccaccccat tgacgtcaat gggagtttgt tttggcacca aaatcaacgg gactttccaa 2040
   aatgtcgtaa caactccgcc ccattgacgc aaatgggcgg taggcgtgta cggtgggagg 2100
   tctatataag cagagctcgt ttagtgaacc gtcagatcgc ctggagacgc catccacgct 2160
   gttttgacct ccatagaaga caccgggacc gatccagcct ggggatctag cctccgcggc 2220
   cgggaacggt gcattggaac gcggattccc cgtgccaaga gtgacgtaag taccgcctat 2280
   agagtctata ggcccacccc cttggcttct tatgcatgct atactgtttt tggcttgggg 2340
   tctatacacc cccgcttcct catgttatag gtgatggtat agcttagcct ataggtgtgg 2400
   gttattgacc attattgacc actcccctat tggtgacgat ctttccatta ctaatccata 2460
   acatggctct ttgccacaac tctctttatt ggctatatgc caatacactg tccttcagag 2520
   actgacacgg actctgtatt tttacaggat ggggtctcat ttattattta caaattcaca 2580
   tatacaacac caccgtcccc agtgcccgca gtttttatta aacataacgt gggatctcca 2640
   cgcgaatctc gggtacgtgt tccggacatg ggctcttctc cggtagcggc ggagctccta 2700
   catccgagcc ctgctcccat gcctccagcg actcatggtc gctcggcagc tccttgctcc 2760
   taacagtgga ggccagactt aggcacagca cgatgcccac caccaccagt gtgccgcaca 2820
   aggccgtggc ggtagggtat gtgtctgaaa atgagctcgg ggagcgggct tgcaccgctg 2880
   acgcatttgg aagacttaag gcagcggcag aagaagatgc aggcagctga gttgttgtgt 2940
   tctgataaga gtcagaggta actcccgttg cggtgctgtt aacggtggag ggcagtgtag 3000
   tctgagcagt actcgttgct gccgcgcgcg ccaccagaca taatagctga cagactaaca 3060
   gactgttcct ttccatgggt cttttctgca gtcaccgtcc ttgacacgat cggatcccgg 3120
   gtaccgagct cggatccagt acccttcacc gacagagctc ttcatcatgg attctatatt 3180
   atcaaaacag ctggttgaca agactggttt tgttagagtg ccaatcaagc attttgactg 3240
   tacaatgcta actctggcac ttccaacatt tgatgtttcc aagatggtag atagaattac 3300
   catagacttc aatctggatg atatacaagg agcatctgaa ataggctcaa ctttgctacc 3360
   ctccatgtcg atagatgtgg aagatatggc caattttgtt cacgatttca cctttggcca 3420
   cttagctgac aagactgaca gactgttaat gcgtgagttt cccatgatga atgacgggtt 3480
   tgatcatttg agccctgaca tgatcattaa aactacatct ggcatgtaca acatcgttga 3540
   gttcaccacc tttaggggag atgaaagagg tgcattccag gctgccatga ctaaactcgc 3600
   taagtatgag gttccttgtg agaacagatc tcagggcagg actgttgttc tttatgttgt 3660
   tagtgcttat cggcatggtg tatggtctaa tctggagcta gaggactctg aagcagagga 3720
   gatggtttat aggtacagac ttgctcttag tgtgatggat gagctaagga ccttgttccc 3780
   agaactgtca tccacagatg aggaactagg gaagactgag agagagttgc tagccatggt 3840
   ctcctccatc caaataaatt ggtcagtcac agaatctgtg tttccaccct tcagcagaga 3900
   aatgtttgac aggtttagat cctcccctcc cgattcagag tatatcacga ggatagtgag 3960
   cagatgccta ataaattctc aagagaaact catcaatagt tccttctttg ctgaagggaa 4020
   tgataaggct ctgagatttt caaaaaacgc tgaagagtgt tccttggcag tagagagagc 4080
   cttaaatcag tatagagcag aagacaacct tagggacctc aatgaccaca agtcaactat 4140
   tcagctgcct ccctggctgt cctatcatga tgtcgatggc aaagatctgt gccctcttca 4200
   gggactagat gtgagagggg accatcccat gtgcaacttg tggagggaag tggtcacctc 4260
   tgcaaaccta gaggagattg agaggatgca cgatgatgca gcagcagaac ttgagtttgc 4320
   cctttcggga gtaaaggaca ggccagatga gagaaacaga taccatagag tccacctaaa 4380
   tatgggctca gatgatagtg tctacatagc tgctttagga gttaatggaa agaagcataa 4440
   agcagacact ttagtgcaac aaatgagaga caggagtaaa cagcctttct ccccagacca 4500
   cgatgtggat cacatatctg aatttctctc tgcatgctct agtgacttgt gggcaacaga 4560
   tgaggacctg tacagccctc tctcttgtga taaagagctt agattggcag cccagaggat 4620
   tcatcagcca tccttgtcag aaaggggttt caatgagatc ataacagagc actacaaatt 4680
   catgggaagt aggataggtt catggtgcca aatggtcagc ttgataggag ctgagctatc 4740
   agcttctgtt aaacaacatg tcaagcctaa ctactttgtg attaaacgac tactaggttc 4800
   tgggattttc ttgctaatca agcccacttc cagcaaaagc catatatttg tgtcttttgc 4860
   aattaagcgc tcttgctggg cctttgatct ctccacttcc agggttttca agccctacat 4920
   agatgctggg gatctgttag ttactgactt tgtttcttat aagctaagca agcttaccaa 4980
   cctctgcaag tgcgtttcat taatggagtc ctccttctca ttctgggcag aagcatttgg 5040
   aattccaagc tggaactttg ttggtgactt gttcaggtct tcagactctg cagcaatgga 5100
   tgcctcatac atgggcaaac tttctttatt aacccttttg gaagacaaag cagcaactga 5160
   agagttacag actattgcaa gatatataat catggagggc tttgtctcgc ccccagaaat 5220
   cccaaaacct cacaagatga cctctaagtt tcctaaggtt ctcaggtcag agctgcaggt 5280
   ttacttatta aactgcttat gcagaactat ccagagaata gcaggtgagc ccttcattct 5340
   taagaagaag gatgggtcta tatcctgggg tggcatgttc aatccttttt cagggcgtcc 5400
   actgcttgat atgcaaccac tcatcagctg ttgttacaat ggttacttta aaaataaaga 5460
   agaagagact gagccttcgt ccctttctgg gatgtataag aaaatcatag aacttgagca 5520
   ccttagacca cagtcagatg ccttcttggg ttacaaagat ccagaacttc ccagaatgca 5580
   tgagttcagt gtttcctact tgaaggaggc ttgcaatcat gctaagctag tcttgaggag 5640
   cctctatgga cagaatttca tggagcagat agacaaccag attattcgag agctcagtgg 5700
   gttgactcta gaaaggttgg ccacacttaa ggccacaagc aactttaatg agaattggta 5760
   tgtctataag gatgtagcag acaaaaacta cacaagggat aaattattag tgaagatgtc 5820
   aaaatatgcc tctgagggaa agagcctagc tatccagaag tttgaggatt gtatgaggca 5880
   gatagagtca caaggatgca tgcatatttg tttgtttaag aaacaacagc atggaggtct 5940
   gagagagatc tatgtgatgg gtgcagagga aagaattgtt caatcggtgg tggagacaat 6000
   agccaggtcc atagggaagt tctttgcttc tgataccctc tgtaaccccc ccaataaagt 6060
   gaaaattcct gagacacatg gcatcagggc ccggaagcaa tgtaaggggc ctgtgtggac 6120
   ttgtgcaaca tcagatgatg caaggaagtg gaaccaaggc cattttgtta caaagtttgc 6180
   cctcatgctg tgtgagttca cctctcctaa atggtggccg ctgatcatta ggggatgctc 6240
   aatgtttacc aggaaaagga tgatgatgaa tttgaattat cttaagatcc tggatggtca 6300
   tcgggagctt gatattagag atgactttgt aatggatctc ttcaaagctt atcatggcga 6360
   ggcagaagtt ccatgggcct ttaaaggcaa aacatatttg gaaaccacaa cagggatgat 6420
   gcagggaata ctgcattata cttcctcact attacacacc attcaccaag aatacatccg 6480
   gtccttgtcc tttaagatat tcaacctgaa ggttgctcct gagatgagca agagcctggt 6540
   ttgtgacatg atgcaaggat cagatgatag tagtatgcta atcagcttcc cagctgatga 6600
   tgagaaggtt cttaccagat gcaaagtggc cgcagctata tgcttccgca tgaagaagga 6660
   gctgggagtg taccttgcca tttacccctc agagaagtcc acagcaaaca cagattttgt 6720
   gatggagtac aattctgaat tttatttcca cacccagcat gttagaccaa cgatcaggtg 6780
   gattgcagct tgttgcagcc tgccagaagt ggaaacacta gtagcccgcc aggaagaggc 6840
   ctctaaccta atgacttcag ttactgaggg aggtgggtca ttctccttag ctgcaatgat 6900
   tcagcaagct cagtgtactc tccattacat gctgatgggc atgggagtgt ctgagctatt 6960
   cttagagtat aagaaggcag tgctgaagtg gaatgaccct ggcctgggtt tcttcctgct 7020
   tgacaatcct tatgcgtgcg gattgggagg tttcagattt aatctcttca aagctatcac 7080
   cagaactgat ttgcagaagc tatatgcttt cttcatgaag aaggtcaagg gctcagctgc 7140
   tagggactgg gcagatgaag atgtcaccat cccagaaacg tgtagcgtga gcccaggtgg 7200
   cgctctaatt cttagctcct ctctaaagtg gggatctagg aagaagtttc agaaattgag 7260
   agaccgtttg aacataccag agaactggat tgaactaata aatgagaatc cagaggtgct 7320
   ctatcgggct cccagaacag gcccagaaat attgttgcgc attgcagaga aagtccatag 7380
   cccaggtgtt gtgtcatcat tgtcttctgg caatgcagtt tgtaaagtca tggcctcagc 7440
   tgtatacttc ttatcagcaa caatttttga ggacactgga cgtcctgagt tcaacttctt 7500
   ggaggattct aagtacagct tgctacaaaa gatggctgca tattctggct ttcatggttt 7560
   caatgatatg gagccagaag atatattatt cttattcccg aatattgagg aattagaatc 7620
   actggattct atagtttaca acaagggaga aatagacatc atcccaagag tcaacatcag 7680
   ggatgcaacc caaaccaggg tcactatctt taatgagcag aagaccctcc ggacatctcc 7740
   agagaagttg gtgtcagaca agtggtttgg gactcagaag agtaggatag gcaaaacaac 7800
   cttcctggct gaatgggaaa agctaaagaa aattgtaaag tggttggaag acactccaga 7860
   agcaactcta gctcacaccc cactgaataa ccatattcaa gttaggaatt tctttgctag 7920
   aatggaaagc aagcctagaa cagtcagaat aacaggagct ccagtaaaga agaggtcagg 7980
   ggttagtaag atagctatgg ttatccgtga caatttctcc cggatgggcc atcttcgagg 8040
   tgtagaagac cttgctggct tcactcgtag tgtgtcagct gaaattctca agcactttct 8100
   attctgtata ctacaaggtc catacagtga gagctataag ctacagctaa tctacagagt 8160
   cctaagctca gtgtcaaacg ttgagataaa ggaatcagat ggtaagacaa aaaccaactt 8220
   gattggaatc cttcagagat ttctagatgg tgatcacgtt gtccccataa ttgaagagat 8280
   gggagccgga acagtgggtg gattcatcaa gagacaacaa tctaaagttg tgcagaacaa 8340
   agtggtctat tatggagttg ggatttggag aggcttcatg gatggatatc aggtccatct 8400
   agagatagaa aatgacatag gacagccccc aaggcttagg aatgtcacaa ctaactgtca 8460
   gagcagccca tgggacctga gtattccaat aaggcaatgg gcagaagaca tgggggtcac 8520
   aaacaaccag gattattctt ctaaatctag caggggggcc agatattgga tgcattcatt 8580
   caggatgcaa ggacctagca agccatttgg atgcccagtt tatattatta agggtgatat 8640
   gtcagatgtc atcagactga gaaaggagga ggtggagatg aaagtacggg gctctactct 8700
   caacttgtac accaagcacc attctcatca ggacctacac attctatctt acactgcatc 8760
   agacaatgat ctcagtccag gcattttcaa gtcaatatca gatgaggggg tggctcaagc 8820
   cctgcaatta tttgagaggg agccaagcaa ctgctgggtg agatgtgagt ctgtagcccc 8880
   aaaatttata tcagccatcc ttgagatatg tgaggggaag agacagataa agggaattaa 8940
   cagaaccaga ctctcagaga ttgtgagaat ttgttctgaa tcttccctaa gatcaaaagt 9000
   cggatctatg ttctcatttg tcgccaatgt cgaggaggcc catgatgttg attatgatgc 9060
   gttaatggat ctaatgatag aggatgccaa gaacaatgca ttcagtcatg ttgttgactg 9120
   catagagttg gatgttagtg gcccttacga gatggagtct tttgatacat ctgatgtcaa 9180
   tctctttggg ccagcccatt acaaggacat cagttcatta tctatgattg ctcatccctt 9240
   aatggataag tttgttgatt atgctatttc taagatgggg agagcctcag ttaggaaagt 9300
   tctagaaaca ggtcggtgct ccagcaaaga ctatgattta tcaaaggttc tcttcagaac 9360
   tctacagaga ccagaagaaa gcattaggat agatgatctg gaattatatg aggagacaga 9420
   tgtggcggat gacatgctag gctaagacca gaagagctct gtcaagggtc aagacaattc 9480
   tgcagatatc cagcacagtg gcggccgctc gaggaattct agatcccacg tcactattgt 9540
   atactctata ttatactcta tgttatactc tgtaatccta ctcaataaac gtgtcacgcc 9600
   tgtgaaaccg tactaagtct cccgtgtctt cttatcacca tcaggtgaca tcctcgccca 9660
   ggctgtcaat catgccggta tcgattccag tagcaccggc cccacgctga caacccactc 9720
   ttgcagcgtt agcagcgccc ctcttaacaa gccgaccccc accagcgtcg cggttactaa 9780
   cactcctctc cccgacctgc agcccaagct tggcgtaatc atggtcatag ctgtttcctg 9840
   tgtgaaattg ttatccgctc acaattccac acaacatacg agccggaagc ataaagtgta 9900
   aagcctgggg tgcctaatga gtgagctaac tcacattaat tgcgttgcgc tcactgcccg 9960
   ctttccagtc gggaaacctg tcgtgccagc tgcattaatg aatcggccaa cgcgcgggga 10020
   gaggcggttt gcgtattggg cgctcttccg cttcctcgct cactgactcg ctgcgctcgg 10080
   tcgttcggct gcggcgagcg gtatcagctc actcaaaggc ggtaatacgg ttatccacag 10140
   aatcagggga taacgcagga aagaacatgt gagcaaaagg ccagcaaaag gccaggaacc 10200
   gtaaaaaggc cgcgttgctg gcgtttttcc ataggctccg cccccctgac gagcatcaca 10260
   aaaatcgacg ctcaagtcag aggtggcgaa acccgacagg actataaaga taccaggcgt 10320
   ttccccctgg aagctccctc gtgcgctctc ctgttccgac cctgccgctt accggatacc 10380
   tgtccgcctt tctcccttcg ggaagcgtgg cgctttctca tagctcacgc tgtaggtatc 10440
   tcagttcggt gtaggtcgtt cgctccaagc tgggctgtgt gcacgaaccc cccgttcagc    10500
   ccgaccgctg cgccttatcc ggtaactatc gtcttgagtc caacccggta agacacgact    10560
   tatcgccact ggcagcagcc actggtaaca ggattagcag agcgaggt                 10608
<210> 26
   <211> 7954
   <212> DNA
   <213> artificial sequence
<220>
   <223> vector
<400> 26
   atgtaggcgg tgctacagag ttcttgaagt ggtggcctaa ctacggctac actagaagaa      60
   cagtatttgg tatctgcgct ctgctgaagc cagttacctt cggaaaaaga gttggtagct     120
   cttgatccgg caaacaaacc accgctggta gcggtggttt ttttgtttgc aagcagcaga     180
   ttacgcgcag aaaaaaagga tctcaagaag atcctttgat cttttctacg gggtctgacg     240
   ctcagtggaa cgaaaactca cgttaaggga ttttggtcat gagattatca aaaaggatct     300
   tcacctagat ccttttaaat taaaaatgaa gttttaaatc aatctaaagt atatatgagt     360
   aaacttggtc tgacagttac caatgcttaa tcagtgaggc acctatctca gcgatctgtc     420
   tatttcgttc atccatagtt gcctgactcc ccgtcgtgta gataactacg atacgggagg     480
   gcttaccatc tggccccagt gctgcaatga taccgcgaga cccacgctca ccggctccag     540
   atttatcagc aataaaccag ccagccggaa gggccgagcg cagaagtggt cctgcaactt     600
   tatccgcctc catccagtct attaattgtt gccgggaagc tagagtaagt agttcgccag     660
   ttaatagttt gcgcaacgtt gttgccattg ctacaggcat cgtggtgtca cgctcgtcgt     720
   ttggtatggc ttcattcagc tccggttccc aacgatcaag gcgagttaca tgatccccca     780
   tgttgtgcaa aaaagcggtt agctccttcg gtcctccgat cgttgtcaga agtaagttgg     840
   ccgcagtgtt atcactcatg gttatggcag cactgcataa ttctcttact gtcatgccat     900
   ccgtaagatg cttttctgtg actggtgagt actcaaccaa gtcattctga gaatagtgta     960
   tgcggcgacc gagttgctct tgcccggcgt caatacggga taataccgcg ccacatagca    1020
   gaactttaaa agtgctcatc attggaaaac gttcttcggg gcgaaaactc tcaaggatct    1080
   taccgctgtt gagatccagt tcgatgtaac ccactcgtgc acccaactga tcttcagcat    1140
   cttttacttt caccagcgtt tctgggtgag caaaaacagg aaggcaaaat gccgcaaaaa    1200
   agggaataag ggcgacacgg aaatgttgaa tactcatact cttccttttt caatattatt    1260
   gaagcattta tcagggttat tgtctcatga gcggatacat atttgaatgt atttagaaaa    1320
   ataaacaaat aggggttccg cgcacttttc cccgaaaagt gccacctgac gtctaagaaa    1380
   ccattattat catgacatta acctataaaa ataggcgtat cacgaggccc tttcgtctcg 1440
   cgcgtttcgg tgatgacggt gaaaacctct gacacatgca gctcccggag acggtcacag 1500
   cttgtctgta agcggatgcc gggagcagac aagcccgtca gggcgcgtca gcgggtgttg 1560
   gcgggtgtcg gggctggctt aactatgcgg catcagagca gattgtactg agagtgcacc 1620
   atatgcggtg tgaaataccg cacagatgcg taaggagaaa ataccgcatc aggcgccatt 1680
   cgccattcag gctgcgcaac tgttgggaag ggcgatcggt gcgggcctct tcgctattac 1740
   gccagctggc gaaaggggga tgtgctgcaa ggcgattaag ttgggtaacg ccagggtttt 1800
   cccagtcacg acgttgtaaa acgacggcca gtgccaagct tgcatgcctg caggtcaatt 1860
   ccctggcatt atgcccagta catgacctta tgggactttc ctacttggca gtacatctac 1920
   gtattagtca tcgctattac catggtgatg cggttttggc agtacatcaa tgggcgtgga 1980
   tagcggtttg actcacgggg atttccaagt ctccacccca ttgacgtcaa tgggagtttg 2040
   ttttggcacc aaaatcaacg ggactttcca aaatgtcgta acaactccgc cccattgacg 2100
   caaatgggcg gtaggcgtgt acggtgggag gtctatataa gcagagctcg tttagtgaac 2160
   cgtcagatcg cctggagacg ccatccacgc tgttttgacc tccatagaag acaccgggac 2220
   cgatccagcc tggggatcta gcctccgcgg ccgggaacgg tgcattggaa cgcggattcc 2280
   ccgtgccaag agtgacgtaa gtaccgccta tagagtctat aggcccaccc ccttggcttc 2340
   ttatgcatgc tatactgttt ttggcttggg gtctatacac ccccgcttcc tcatgttata 2400
   ggtgatggta tagcttagcc tataggtgtg ggttattgac cattattgac cactccccta 2460
   ttggtgacga tctttccatt actaatccat aacatggctc tttgccacaa ctctctttat 2520
   tggctatatg ccaatacact gtccttcaga gactgacacg gactctgtat ttttacagga 2580
   tggggtctca tttattattt acaaattcac atatacaaca ccaccgtccc cagtgcccgc 2640
   agtttttatt aaacataacg tgggatctcc acgcgaatct cgggtacgtg ttccggacat 2700
   gggctcttct ccggtagcgg cggagctcct acatccgagc cctgctccca tgcctccagc 2760
   gactcatggt cgctcggcag ctccttgctc ctaacagtgg aggccagact taggcacagc 2820
   acgatgccca ccaccaccag tgtgccgcac aaggccgtgg cggtagggta tgtgtctgaa 2880
   aatgagctcg gggagcgggc ttgcaccgct gacgcatttg gaagacttaa ggcagcggca 2940
   gaagaagatg caggcagctg agttgttgtg ttctgataag agtcagaggt aactcccgtt 3000
   gcggtgctgt taacggtgga gggcagtgta gtctgagcag tactcgttgc tgccgcgcgc 3060
   gccaccagac ataatagctg acagactaac agactgttcc tttccatggg tcttttctgc 3120
   agtcaccgtc cttgacacga tcggatcccg ggtaccgagc tcggatccag tacccttcac 3180
   cgacagagct cttctaaatg tatgttttat taacaattct aatctcggtt ctggtgtgtg 3240
   aagcggttat tagagtgtct ctaagctcca caagagaaga gacctgcttt ggtgactcca 3300
   ctaacccaga gatgattgaa ggagcttggg attcactcag agaggaggag atgccggagg 3360
   agctctcctg ttctatatca ggcataagag aggttaagac ctcaagccag gagttataca 3420
   gggcattaaa agccatcatt gctgctgatg gcttgaacaa catcacctgc catggtaagg 3480
   atcctgagga caagatttcc ctcataaagg gtcctcctca caaaaagcgg gtggggatag 3540
   ttcggtgtga gagacgaaga gatgctaagc aaatagggag agaaaccatg gcagggattg 3600
   caatgacagt ccttccagcc ttagcagttt ttgctttggc acctgttgtt tttgctgaag 3660
   acccccatct cagaaacaga ccagggaagg ggcacaacta cattgacggg atgactcagg 3720
   aggatgccac atgcaaacct gtgacatatg ctggggcatg tagcagtttt gatgtcttgc 3780
   ttgaaaaggg aaaatttccc cttttccagt cgtatgctca tcatagaact ctactagagg 3840
   cagttcacga caccatcatt gcaaaggctg atccacctag ctgtgacctt ctgagtgctc 3900
   atgggaaccc ctgcatgaaa gagaaactcg tgatgaagac acactgtcca aatgactacc 3960
   agtcagctca ttacctcaac aatgacggga aaatggcttc agtcaagtgc cctcctaagt 4020
   atgagctcac tgaggactgc aacttttgta ggcagatgac aggtgctagc ctgaagaagg 4080
   ggtcttatcc tctccaagac ttgttttgtc agtcaagtga ggatgatgga tcaaaattaa 4140
   aaacaaaaat gaaaggggtc tgcgaagtgg gggttcaagc actcaaaaag tgtgatggcc 4200
   aactcagcac tgcacatgag gttgtgccct ttgcagtgtt taagaactca aagaaggttt 4260
   atcttgataa gcttgacctt aagactgagg agaatctgct accagactca tttgtctgtt 4320
   tcgagcataa gggacagtac aaaggaacaa tggactctgg tcagactaag agggagctca 4380
   aaagctttga tatctctcag tgccccaaga ttggaggaca tggtagtaag aagtgcactg 4440
   gggacgcagc attttgctct gcttatgagt gcactgctca gtacgccaat gcctattgtt 4500
   cacatgctaa tgggtcaggg attgtgcaga tacaagtatc aggggtctgg aagaagcctt 4560
   tatgtgtagg gtatgagaga gtggttgtga agagagaact ctctgccaag cccatccaga 4620
   gagttgagcc ttgcacaact tgtataacca aatgtgagcc tcatggattg gttgtccgat 4680
   caacagggtt caagatatca tcagcagttg cttgtgctag cggagtttgc gtcacaggat 4740
   cacagagtcc ttccaccgag attacactca agtatccagg gatatcccag tcttctgggg 4800
   gggacatagg ggttcacatg gcacacgatg atcagtcagt tagctccaaa atagtagctc 4860
   actgccctcc ccaggacccg tgcttagtgc atgactgcat agtgtgtgct catggcctga 4920
   taaattacca gtgtcacact gctctcagtg cctttgttgt tgtgtttgta ttcagttcta 4980
   ttgcaataat ttgtttagct attctctata gggtgcttaa gtgcctgaag attgccccaa 5040
   ggaaagttct gaatccacta atgtggatca cagccttcat cagatggata tataagaaga 5100
   tggttgccag agtggcagac aacattaatc aagtgaacag ggaaatagga tggatggaag 5160
   gaggtcagtt ggttctaggg aaccctgccc ctattcctcg tcatgcccca atcccacgtt 5220
   atagcacata cctgatgtta ttattgattg tctcatatgc atcagcatgt tcagaactga 5280
   ttcaggcaag ctccagaatc accacttgct ctacagaggg tgttaacacc aagtgtagac 5340
   tgtctggcac agcattgatc agagcagggt cagttggggc agaggcttgt ttgatgttga 5400
   agggggtcaa ggaagatcaa accaagttct taaagataaa aactgtctca agtgagctat 5460
   catgcaggga gggccagagt tattggactg ggtcctttag ccctaaatgt ttgagctcaa 5520
   ggagatgcca ccttgtcggg gaatgccatg tgaataggtg tctgtcttgg agggacaatg 5580
   aaacttcagc agagttttca tttgttgggg aaagcacgac catgcgagag aataagtgtt 5640
   ttgagcaatg tggaggatgg gggtgtgggt gtttcaatgt gaacccatct tgcttatttg 5700
   tgcacacgta tctgcagtca gttagaaaag aggcccttag agtttttaac tgtatcgact 5760
   gggtgcataa actcactcta gagatcacag actttgatgg ctctgtttca acaatagact 5820
   tgggagcatc atctagccgt ttcacaaact ggggttcagt tagcctctca ctggacgcag 5880
   agggcatttc aggctcaaat agcttttctt tcattgagag cccaggcaaa gggtatgcaa 5940
   ttgttgatga gccattctca gaaattcctc ggcaagggtt cttgggggag atcaggtgca 6000
   attcagagtc ctcagtcctg agtgctcatg aatcatgcct tagggcacca aaccttatct 6060
   catacaagcc catgatagat caattggagt gcacaacaaa tctgattgat ccctttgttg 6120
   tctttgagag gggttctctg ccacagacaa ggaatgacaa aacctttgca gcttcaaaag 6180
   gaaatagagg tgttcaagct ttctctaagg gctctgtaca agctgatcta actctgatgt 6240
   ttgacaattt tgaggtggac tttgtgggag cagccgtatc ttgtgatgcc gccttcttaa 6300
   atttgacagg ttgctattct tgcaatgcag gggccagggt ctgcctgtct atcacatcca 6360
   caggaactgg atccctctct gcccacaata aggatgggtc tctgcatata gtccttccat 6420
   cagagaatgg aacaaaagac cagtgtcaga tactacactt cactgtgcct gaagtagagg 6480
   aggagtttat gtactcttgt gatggagatg agcggcctct gttggtgaag gggaccctga 6540
   tagccattga tccatttgat gataggcggg aagcaggggg ggaatcaaca gttgtgaatc 6600
   caaaatctgg atcttggaat ttctttgact ggttttctgg actcatgagt tggtttggag 6660
   ggcctcttaa aactatactc ctcatttgcc tgtatgttgc attatcaatt gggctctttt 6720
   tcctccttat atatcttgga agaacaggcc tctctaaaat gtggcttgct gccactaaga 6780
   aggcctcata gatcaggaag agctctgtca agggtcaaga caattctgca gatatccagc 6840
   acagtggcgg ccgctcgagg aattctagat cccacgtcac tattgtatac tctatattat 6900
   actctatgtt atactctgta atcctactca ataaacgtgt cacgcctgtg aaaccgtact 6960
   aagtctcccg tgtcttctta tcaccatcag gtgacatcct cgcccaggct gtcaatcatg 7020
   ccggtatcga ttccagtagc accggcccca cgctgacaac ccactcttgc agcgttagca 7080
   gcgcccctct taacaagccg acccccacca gcgtcgcggt tactaacact cctctccccg 7140
   acctgcagcc caagcttggc gtaatcatgg tcatagctgt ttcctgtgtg aaattgttat 7200
   ccgctcacaa ttccacacaa catacgagcc ggaagcataa agtgtaaagc ctggggtgcc 7260
   taatgagtga gctaactcac attaattgcg ttgcgctcac tgcccgcttt ccagtcggga 7320
   aacctgtcgt gccagctgca ttaatgaatc ggccaacgcg cggggagagg cggtttgcgt 7380
   attgggcgct cttccgcttc ctcgctcact gactcgctgc gctcggtcgt tcggctgcgg 7440
   cgagcggtat cagctcactc aaaggcggta atacggttat ccacagaatc aggggataac 7500
   gcaggaaaga acatgtgagc aaaaggccag caaaaggcca ggaaccgtaa aaaggccgcg 7560
   ttgctggcgt ttttccatag gctccgcccc cctgacgagc atcacaaaaa tcgacgctca 7620
   agtcagaggt ggcgaaaccc gacaggacta taaagatacc aggcgtttcc ccctggaagc 7680
   tccctcgtgc gctctcctgt tccgaccctg ccgcttaccg gatacctgtc cgcctttctc 7740
   ccttcgggaa gcgtggcgct ttctcatagc tcacgctgta ggtatctcag ttcggtgtag 7800
   gtcgttcgct ccaagctggg ctgtgtgcac gaaccccccg ttcagcccga ccgctgcgcc 7860
   ttatccggta actatcgtct tgagtccaac ccggtaagac acgacttatc gccactggca 7920
   gcagccactg gtaacaggat tagcagagcg aggt 7954
<210> 27
   <211> 7954
   <212> DNA
   <213> artificial sequence
<220>
   <223> vector
<400> 27
   atgtaggcgg tgctacagag ttcttgaagt ggtggcctaa ctacggctac actagaagaa      60
   cagtatttgg tatctgcgct ctgctgaagc cagttacctt cggaaaaaga gttggtagct     120
   cttgatccgg caaacaaacc accgctggta gcggtggttt ttttgtttgc aagcagcaga     180
   ttacgcgcag aaaaaaagga tctcaagaag atcctttgat cttttctacg gggtctgacg     240
   ctcagtggaa cgaaaactca cgttaaggga ttttggtcat gagattatca aaaaggatct     300
   tcacctagat ccttttaaat taaaaatgaa gttttaaatc aatctaaagt atatatgagt     360
   aaacttggtc tgacagttac caatgcttaa tcagtgaggc acctatctca gcgatctgtc     420
   tatttcgttc atccatagtt gcctgactcc ccgtcgtgta gataactacg atacgggagg 480
   gcttaccatc tggccccagt gctgcaatga taccgcgaga cccacgctca ccggctccag 540
   atttatcagc aataaaccag ccagccggaa gggccgagcg cagaagtggt cctgcaactt 600
   tatccgcctc catccagtct attaattgtt gccgggaagc tagagtaagt agttcgccag 660
   ttaatagttt gcgcaacgtt gttgccattg ctacaggcat cgtggtgtca cgctcgtcgt 720
   ttggtatggc ttcattcagc tccggttccc aacgatcaag gcgagttaca tgatccccca 780
   tgttgtgcaa aaaagcggtt agctccttcg gtcctccgat cgttgtcaga agtaagttgg 840
   ccgcagtgtt atcactcatg gttatggcag cactgcataa ttctcttact gtcatgccat 900
   ccgtaagatg cttttctgtg actggtgagt actcaaccaa gtcattctga gaatagtgta 960
   tgcggcgacc gagttgctct tgcccggcgt caatacggga taataccgcg ccacatagca 1020
   gaactttaaa agtgctcatc attggaaaac gttcttcggg gcgaaaactc tcaaggatct 1080
   taccgctgtt gagatccagt tcgatgtaac ccactcgtgc acccaactga tcttcagcat 1140
   cttttacttt caccagcgtt tctgggtgag caaaaacagg aaggcaaaat gccgcaaaaa 1200
   agggaataag ggcgacacgg aaatgttgaa tactcatact cttccttttt caatattatt 1260
   gaagcattta tcagggttat tgtctcatga gcggatacat atttgaatgt atttagaaaa 1320
   ataaacaaat aggggttccg cgcacatttc cccgaaaagt gccacctgac gtctaagaaa 1380
   ccattattat catgacatta acctataaaa ataggcgtat cacgaggccc tttcgtctcg 1440
   cgcgtttcgg tgatgacggt gaaaacctct gacacatgca gctcccggag acggtcacag 1500
   cttgtctgta agcggatgcc gggagcagac aagcccgtca gggcgcgtca gcgggtgttg 1560
   gcgggtgtcg gggctggctt aactatgcgg catcagagca gattgtactg agagtgcacc 1620
   atatgcggtg tgaaataccg cacagatgcg taaggagaaa ataccgcatc aggcgccatt 1680
   cgccattcag gctgcgcaac tgttgggaag ggcgatcggt gcgggcctct tcgctattac 1740
   gccagctggc gaaaggggga tgtgctgcaa ggcgattaag ttgggtaacg ccagggtttt 1800
   cccagtcacg acgttgtaaa acgacggcca gtgccaagct tgcatgcctg caggtcaatt 1860
   ccctggcatt atgcccagta catgacctta tgggactttc ctacttggca gtacatctac 1920
   gtattagtca tcgctattac catggtgatg cggttttggc agtacatcaa tgggcgtgga 1980
   tagcggtttg actcacgggg atttccaagt ctccacccca ttgacgtcaa tgggagtttg 2040
   ttttggcacc aaaatcaacg ggactttcca aaatgtcgta acaactccgc cccattgacg 2100
   caaatgggcg gtaggcgtgt acggtgggag gtctatataa gcagagctcg tttagtgaac 2160
   cgtcagatcg cctggagacg ccatccacgc tgttttgacc tccatagaag acaccgggac 2220
   cgatccagcc tggggatcta gcctccgcgg ccgggaacgg tgcattggaa cgcggattcc 2280
   ccgtgccaag agtgacgtaa gtaccgccta tagagtctat aggcccaccc ccttggcttc 2340
   ttatgcatgc tatactgttt ttggcttggg gtctatacac ccccgcttcc tcatgttata 2400
   ggtgatggta tagcttagcc tataggtgtg ggttattgac cattattgac cactccccta 2460
   ttggtgacga tctttccatt actaatccat aacatggctc tttgccacaa ctctctttat 2520
   tggctatatg ccaatacact gtccttcaga gactgacacg gactctgtat ttttacagga 2580
   tggggtctca tttattattt acaaattcac atatacaaca ccaccgtccc cagtgcccgc 2640
   agtttttatt aaacataacg tgggatctcc acgcgaatct cgggtacgtg ttccggacat 2700
   gggctcttct ccggtagcgg cggagctcct acatccgagc cctgctccca tgcctccagc 2760
   gactcatggt cgctcggcag ctccttgctc ctaacagtgg aggccagact taggcacagc 2820
   acgatgccca ccaccaccag tgtgccgcac aaggccgtgg cggtagggta tgtgtctgaa 2880
   aatgagctcg gggagcgggc ttgcaccgct gacgcatttg gaagacttaa ggcagcggca 2940
   gaagaagatg caggcagctg agttgttgtg ttctgataag agtcagaggt aactcccgtt 3000
   gcggtgctgt taacggtgga gggcagtgta gtctgagcag tactcgttgc tgccgcgcgc 3060
   gccaccagac ataatagctg acagactaac agactgttcc tttccatggg tcttttctgc 3120
   agtcaccgtc cttgacacga tcggatcccg ggtaccgagc tcggatccag tacccttcac 3180
   cgacagagct cttctaaatg tatgttttat taacaattct aatctcggtt ctggtgtgtg 3240
   aagcggttat tagagtgtct ctaagctcca caagagaaga gacctgcttt ggtgactcca 3300
   ctaacccaga gatgattgaa ggagcttggg attcactcag agaggaggag atgccggagg 3360
   agctctcctg ttctatatca ggcataagag aggttaagac ctcaagccag gagttataca 3420
   gggcattaaa agccatcatt gctgctgatg gcttgaacaa catcacctgc catggtaagg 3480
   atcctgagga caagatttcc ctcataaagg gtcctcctca caaaaagcgg gtggggatag 3540
   ttcggtgtga gagacgaaga gatgctaagc aaatagggag agaaaccatg gcagggattg 3600
   caatgacagt ccttccagcc ttagcagttt ttgctttggc acctgttgtt tttgctgaag 3660
   acccccatct cagaaacaga ccagggaagg ggcacaacta cattgacggg atgactcagg 3720
   aggatgccac atgcaaacct gtgacatatg ctggggcatg tagcagtttt gatgtcttgc 3780
   ttgaaaaggg aaaatttccc cttttccagt cgtatgctca tcatagaact ctactagagg 3840
   cagttcacga caccatcatt gcaaaggctg atccacctag ctgtgacctt ctgagtgctc 3900
   atgggaaccc ctgcatgaaa gagaaactcg tgatgaagac acactgtcca aatgactacc 3960
   agtcagctca ttacctcaac aatgacggga aaatggcttc agtcaagtgc cctcctaagt 4020
   atgagctcac tgaggactgc aacttttgta ggcagatgac aggtgctagc ctgaagaagg 4080
   ggtcttatcc tctccaagac ttgttttgtc agtcaagtga ggatgatgga tcaaaattaa 4140
   aaacaaaaat gaaaggggtc tgcgaagtgg gggttcaagc actcaaaaag tgtgatggcc 4200
   aactcagcac tgcacatgag gttgtgccct ttgcagtgtt taagaactca aagaaggttt 4260
   atcttgataa gcttgacctt aagactgagg agaatctgct accagactca tttgtctgtt 4320
   tcgagcataa gggacagtac aaaggaacaa tggactctgg tcagactaag agggagctca 4380
   aaagctttga tatctctcag tgccccaaga ttggaggaca tggtagtaag aagtgcactg 4440
   gggacgcagc attttgctct gcttatgagt gcactgctca gtacgccaat gcctattgtt 4500
   cacatgctaa tgggtcaggg attgtgcaga tacaagtatc aggggtctgg aagaagcctt 4560
   tatgtgtagg gtatgagaga gtggttgtga agagagaact ctctgccaag cccatccaga 4620
   gagttgagcc ttgcacaact tgtataacca aatgtgagcc tcatggattg gttgtccgat 4680
   caacagggtt caagatatca tcagcagttg cttgtgctag cggagtttgc gtcacaggat 4740
   cacagagtcc ttccaccgag attacactca agtatccagg gatatcccag tcttctgggg 4800
   gggacatagg ggttcacatg gcacacgatg atcagtcagt tagctccaaa atagtagctc 4860
   actgccctcc ccaggacccg tgcttagtgc atgactgcat agtgtgtgct catggcctga 4920
   taaattacca gtgtcacact gctctcagtg cctttgttgt tgtgtttgta ttcagttcta 4980
   ttgcaataat ttgtttagct attctctata gggtgcttaa gtgcctgaag attgccccaa 5040
   ggaaagttct gaatccacta atgtggatca cagccttcat cagatggata tataagaaga 5100
   tggttgccag agtggcagac aacattaatc aagtgaacag ggaaatagga tggatggaag 5160
   gaggtcagtt ggttctaggg aaccctgccc ctattcctcg tcatgcccca atcccacgtt 5220
   atagcacata cctgatgtta ttattgattg tctcatatgc atcagcatgt tcagaactga 5280
   ttcaggcaag ctccagaatc accacttgct ctacagaggg tgttaacacc aagtgtagac 5340
   tgtctggcac agcattgatc agagcagggt cagttggggc agaggcttgt ttgatgttga 5400
   agggggtcaa ggaagatcaa accaagttct taaagataaa aactgtctca agtgagctat 5460
   catgcaggga gggccagagt tattggactg ggtcctttag ccctaaatgt ttgagctcaa 5520
   ggagatgcca ccttgtcggg gaatgccatg tgaataggtg tctgtcttgg agggacaatg 5580
   aaacttcagc agagttttca tttgttgggg aaagcacgac catgcgagag aataagtgtt 5640
   ttgagcaatg tggaggatgg gggtgtgggt gtttcaatgt gaacccatct tgcttatttg 5700
   tgcacacgta tctgcagtca gttagaaaag aggcccttag agtttttaac tgtatcgact 5760
   gggtgcataa actcactcta gagatcacag actttgatgg ctctgtttca acaatagact 5820
   tgggagcatc atctagccgt ttcacaaact ggggttcagt tagcctctca ctggacgcag 5880
   agggcatttc aggctcaaat agcttttctt tcattgagag cccaggcaaa gggtatgcaa 5940
   ttgttgatga gccattctca gaaattcctc ggcaagggtt cttgggggag atcaggtgca 6000
   attcagagtc ctcagtcctg agtgctcatg aatcatgcct tagggcacca aaccttatct 6060
   catacaagcc catgatagat caattggagt gcacaacaaa tctgattgat ccctttgttg 6120
   tctttgagag gggttctctg ccacagacaa ggaatgacaa aacctttgca gcttcaaaag 6180
   gaaatagagg tgttcaagct ttctctaagg gctctgtaca agctgatcta actctgatgt 6240
   ttgacaattt tgaggtggac tttgtgggag cagccgtatc ttgtgatgcc gccttcttaa 6300
   atttgacagg ttgctattct tgcaatgcag gggccagggt ctgcctgtct atcacatcca 6360
   caggaactgg atccctctct gcccacaata aggatgggtc tctgcatata gtccttccat 6420
   cagagaatgg aacaaaagac cagtgtcaga tactacactt cactgtgcct gaagtagagg 6480
   aggagtttat gtactcttgt gatggagatg agcggcctct gttggtgaag gggaccctga 6540
   tagccattga tccatttgat gataggcggg aagcaggggg ggaatcaaca gttgtgaatc 6600
   caaaatctgg atcttggaat ttctttgact ggttttctgg actcatgagt tggtttggag 6660
   ggcctcttaa aactatactc ctcatttgcc tgtatgttgc attatcaatt gggctctttt 6720
   tcctccttat atatcttgga agaacaggcc tctctaaaat gtggcttgct gccactaaga 6780
   aggcctcata gatcaggaag agctctgtca agggtcaaga caattctgca gatatccagc 6840
   acagtggcgg ccgctcgagg aattctagat cccacgtcac tattgtatac tctatattat 6900
   actctatgtt atactctgta atcctactca ataaacgtgt cacgcctgtg aaaccgtact 6960
   aagtctcccg tgtcttctta tcaccatcag gtgacatcct cgcccaggct gtcaatcatg 7020
   ccggtatcga ttccagtagc accggcccca cgctgacaac ccactcttgc agcgttagca 7080
   gcgcccctct taacaagccg acccccacca gcgtcgcggt tactaacact cctctccccg 7140
   acctgcagcc caagcttggc gtaatcatgg tcatagctgt ttcctgtgtg aaattgttat 7200
   ccgctcacaa ttccacacaa catacgagcc ggaagcataa agtgtaaagc ctggggtgcc 7260
   taatgagtga gctaactcac attaattgcg ttgcgctcac tgcccgcttt ccagtcggga 7320
   aacctgtcgt gccagctgca ttaatgaatc ggccaacgcg cggggagagg cggtttgcgt 7380
   attgggcgct cttccgcttc ctcgctcact gactcgctgc gctcggtcgt tcggctgcgg 7440
   cgagcggtat cagctcactc aaaggcggta atacggttat ccacagaatc aggggataac 7500
   gcaggaaaga acatgtgagc aaaaggccag caaaaggcca ggaaccgtaa aaaggccgcg 7560
   ttgctggcgt ttttccatag gctccgcccc cctgacgagc atcacaaaaa tcgacgctca 7620
   agtcagaggt ggcgaaaccc gacaggacta taaagatacc aggcgtttcc ccctggaagc 7680
   tccctcgtgc gctctcctgt tccgaccctg ccgcttaccg gatacctgtc cgcctttctc 7740
   ccttcgggaa gcgtggcgct ttctcatagc tcacgctgta ggtatctcag ttcggtgtag 7800
   gtcgttcgct ccaagctggg ctgtgtgcac gaaccccccg ttcagcccga ccgctgcgcc 7860
   ttatccggta actatcgtct tgagtccaac ccggtaagac acgacttatc gccactggca 7920
   gcagccactg gtaacaggat tagcagagcg aggt 7954
<210> 28
   <211> 7954
   <212> DNA
   <213> artificial sequence
<220>
   <223> vector
<400> 28
   atgtaggcgg tgctacagag ttcttgaagt ggtggcctaa ctacggctac actagaagaa      60
   cagtatttgg tatctgcgct ctgctgaagc cagttacctt cggaaaaaga gttggtagct     120
   cttgatccgg caaacaaacc accgctggta gcggtggttt ttttgtttgc aagcagcaga     180
   ttacgcgcag aaaaaaagga tctcaagaag atcctttgat cttttctacg gggtctgacg     240
   ctcagtggaa cgaaaactca cgttaaggga ttttggtcat gagattatca aaaaggatct     300
   tcacctagat ccttttaaat taaaaatgaa gttttaaatc aatctaaagt atatatgagt     360
   aaacttggtc tgacagttac caatgcttaa tcagtgaggc acctatctca gcgatctgtc     420
   tatttcgttc atccatagtt gcctgactcc ccgtcgtgta gataactacg atacgggagg     480
   gcttaccatc tggccccagt gctgcaatga taccgcgaga cccacgctca ccggctccag     540
   atttatcagc aataaaccag ccagccggaa gggccgagcg cagaagtggt cctgcaactt     600
   tatccgcctc catccagtct attaattgtt gccgggaagc tagagtaagt agttcgccag     660
   ttaatagttt gcgcaacgtt gttgccattg ctacaggcat cgtggtgtca cgctcgtcgt     720
   ttggtatggc ttcattcagc tccggttccc aacgatcaag gcgagttaca tgatccccca     780
   tgttgtgcaa aaaagcggtt agctccttcg gtcctccgat cgttgtcaga agtaagttgg     840
   ccgcagtgtt atcactcatg gttatggcag cactgcataa ttctcttact gtcatgccat     900
   ccgtaagatg cttttctgtg actggtgagt actcaaccaa gtcattctga gaatagtgta     960
   tgcggcgacc gagttgctct tgcccggcgt caatacggga taataccgcg ccacatagca    1020
   gaactttaaa agtgctcatc attggaaaac gttcttcggg gcgaaaactc tcaaggatct    1080
   taccgctgtt gagatccagt tcgatgtaac ccactcgtgc acccaactga tcttcagcat    1140
   cttttacttt caccagcgtt tctgggtgag caaaaacagg aaggcaaaat gccgcaaaaa    1200
   agggaataag ggcgacacgg aaatgttgaa tactcatact cttccttttt caatattatt    1260
   gaagcattta tcagggttat tgtctcatga gcggatacat atttgaatgt atttagaaaa 1320
   ataaacaaat aggggttccg cgcacttttc cccgaaaagt gccacctgac gtctaagaaa 1380
   ccattattat catgacatta acctataaaa ataggcgtat cacgaggccc tttcgtctcg 1440
   cgcgtttcgg tgatgacggt gaaaacctct gacacatgca gctcccggag acggtcacag 1500
   cttgtctgta agcggatgcc gggagcagac aagcccgtca gggcgcgtca gcgggtgttg 1560
   gcgggtgtcg gggctggctt aactatgcgg catcagagca gattgtactg agagtgcacc 1620
   atatgcggtg tgaaataccg cacagatgcg taaggagaaa ataccgcatc aggcgccatt 1680
   cgccattcag gctgcgcaac tgttgggaag ggcgatcggt gcgggcctct tcgctattac 1740
   gccagctggc gaaaggggga tgtgctgcaa ggcgattaag ttgggtaacg ccagggtttt 1800
   cccagtcacg acgttgtaaa acgacggcca gtgccaagct tgcatgcctg caggtcaatt 1860
   ccctggcatt atgcccagta catgacctta tgggactttc ctacttggca gtacatctac 1920
   gtattagtca tcgctattac catggtgatg cggttttggc agtacatcaa tgggcgtgga 1980
   tagcggtttg actcacgggg atttccaagt ctccacccca ttgacgtcaa tgggagtttg 2040
   ttttggcacc aaaatcaacg ggactttcca aaatgtcgta acaactccgc cccattgacg 2100
   caaatgggcg gtaggcgtgt acggtgggag gtctatataa gcagagctcg tttagtgaac 2160
   cgtcagatcg cctggagacg ccatccacgc tgttttgacc tccatagaag acaccgggac 2220
   cgatccagcc tggggatcta gcctccgcgg ccgggaacgg tgcattggaa cgcggattcc 2280
   ccgtgccaag agtgacgtaa gtaccgccta tagagtctat aggcccaccc ccttggcttc 2340
   ttatgcatgc tatactgttt ttggcttggg gtctatacac ccccgcttcc tcatgttata 2400
   ggtgatggta tagcttagcc tataggtgtg ggttattgac cattattgac cactccccta 2460
   ttggtgacga tctttccatt actaatccat aacatggctc tttgccacaa ctctctttat 2520
   tggctatatg ccaatacact gtccttcaga gactgacacg gactctgtat ttttacagga 2580
   tggggtctca tttattattt acaaattcac atatacaaca ccaccgtccc cagtgcccgc 2640
   agtttttatt aaacataacg tgggatctcc acgcgaatct cgggtacgtg ttccggacat 2700
   gggctcttct ccggtagcgg cggagctcct acatccgagc cctgctccca tgcctccagc 2760
   gactcatggt cgctcggcag ctccttgctc ctaacagtgg aggccagact taggcacagc 2820
   acgatgccca ccaccaccag tgtgccgcac aaggccgtgg cggtagggta tgtgtctgaa 2880
   aatgagctcg gggagcgggc ttgcaccgct gacgcatttg gaagacttaa ggcagcggca 2940
   gaagaagatg caggcagctg agttgttgtg ttctgataag agtcagaggt aactcccgtt 3000
   gcggtgctgt taacggtgga gggcagtgta gtctgagcag tactcgttgc tgccgcgcgc 3060
   gccaccagac ataatagctg acagactaac agactgttcc tttccatggg tcttttctgc    3120
   agtcaccgtc cttgacacga tcggatcccg ggtaccgagc tcggatccag tacccttcac     3180
   cgacagagct cttctaaatg tatgttttat taacaattct aatctcggtt ctggtgtgtg 3240
   aagcggttat tagagtgtct ctaagctcca caagagaaga gacctgcttt ggtgactcca 3300
   ctaacccaga gatgattgaa ggagcttggg attcactcag agaggaggag atgccggagg 3360
   agctctcctg ttctatatca ggcataagag aggttaagac ctcaagccag gagttataca 3420
   gggcattaaa agccatcatt gctgctgatg gcttgaacaa catcacctgc catggtaagg 3480
   atcctgagga caagatttcc ctcataaagg gtcctcctca caaaaagcgg gtggggatag 3540
   ttcggtgtga gagacgaaga gatgctaagc aaatagggag agaaaccatg gcagggattg 3600
   caatgacagt ccttccagcc ttagcagttt ttgctttggc acctgttgtt tttgctgaag 3660
   acccccatct cagaaacaga ccagggaagg ggcacaacta cattgacggg atgactcagg 3720
   aggatgccac atgcaaacct gtgacatatg ctggggcatg tagcagtttt gatgtcttgc 3780
   ttgaaaaggg aaaatttccc cttttccagt cgtatgctca tcatagaact ctactagagg 3840
   cagttcacga caccatcatt gcaaaggctg atccacctag ctgtgacctt ctgagtgctc 3900
   atgggaaccc ctgcatgaaa gagaaactcg tgatgaagac acactgtcca aatgactacc 3960
   agtcagctca ttacctcaac aatgacggga aaatggcttc agtcaagtgc cctcctaagt 4020
   atgagctcac tgaggactgc aacttttgta ggcagatgac aggtgctagc ctgaagaagg 4080
   ggtcttatcc tctccaagac ttgttttgtc agtcaagtga ggatgatgga tcaaaattaa 4140
   aaacaaaaat gaaaggggtc tgcgaagtgg gggttcaagc actcaaaaag tgtgatggcc 4200
   aactcagcac tgcacatgag gttgtgccct ttgcagtgtt taagaactca aagaaggttt 4260
   atcttgataa gcttgacctt aagactgagg agaatctgct accagactca tttgtctgtt 4320
   tcgagcataa gggacagtac aaaggaacaa tggactctgg tcagactaag agggagctca 4380
   aaagctttga tatctctcag tgccccaaga ttggaggaca tggtagtaag aagtgcactg 4440
   gggacgcagc attttgctct gcttatgagt gcactgctca gtacgccaat gcctattgtt 4500
   cacatgctaa tgggtcaggg attgtgcaga tacaagtatc aggggtctgg aagaagcctt 4560
   tatgtgtagg gtatgagaga gtggttgtga agagagaact ctctgccaag cccatccaga 4620
   gagttgagcc ttgcacaact tgtataacca aatgtgagcc tcatggattg gttgtccgat 4680
   caacagggtt caagatatca tcagcagttg cttgtgctag cggagtttgc gtcacaggat 4740
   cacagagtcc ttccaccgag attacactca agtatccagg gatatcccag tcttctgggg 4800
   gggacatagg ggttcacatg gcacacgatg atcagtcagt tagctccaaa atagtagctc 4860
   actgccctcc ccaggacccg tgcttagtgc atgactgcat agtgtgtgct catggcctga 4920
   taaattacca gtgtcacact gctctcagtg cctttgttgt tgtgtttgta ttcagttcta 4980
   ttgcaataat ttgtttagct attctctata gggtgcttaa gtgcctgaag attgccccaa 5040
   ggaaagttct gaatccacta atgtggatca cagccttcat cagatggata tataagaaga 5100
   tggttgccag agtggcagac aacattaatc aagtgaacag ggaaatagga tggatggaag 5160
   gaggtcagtt ggttctaggg aaccctgccc ctattcctcg tcatgcccca atcccacgtt 5220
   atagcacata cctgatgtta ttattgattg tctcatatgc atcagcatgt tcagaactga 5280
   ttcaggcaag ctccagaatc accacttgct ctacagaggg tgttaacacc aagtgtagac 5340
   tgtctggcac agcattgatc agagcagggt cagttggggc agaggcttgt ttgatgttga 5400
   agggggtcaa ggaagatcaa accaagttct taaagataaa aactgtctca agtgagctat 5460
   catgcaggga gggccagagt tattggactg ggtcctttag ccctaaatgt ttgagctcaa 5520
   ggagatgcca ccttgtcggg gaatgccatg tgaataggtg tctgtcttgg agggacaatg 5580
   aaacttcagc agagttttca tttgttgggg aaagcacgac catgcgagag aataagtgtt 5640
   ttgagcaatg tggaggatgg gggtgtgggt gtttcaatgt gaacccatct tgcttatttg 5700
   tgcacacgta tctgcagtca gttagaaaag aggcccttag agtttttaac tgtatcgact 5760
   gggtgcataa actcactcta gagatcacag actttgatgg ctctgtttca acaatagact 5820
   tgggagcatc atctagccgt ttcacaaact ggggttcagt tagcctctca ctggacgcag 5880
   agggcatttc aggctcaaat agcttttctt tcattgagag cccaggcaaa gggtatgcaa 5940
   ttgttgatga gccattctca gaaattcctc ggcaagggtt cttgggggag atcaggtgca 6000
   attcagagtc ctcagtcctg agtgctcatg aatcatgcct tagggcacca aaccttatct 6060
   catacaagcc catgatagat caattggagt gcacaacaaa tctgattgat ccctttgttg 6120
   tctttgagag gggttctctg ccacagacaa ggaatgacaa aacctttgca gcttcaaaag 6180
   gaaatagagg tgttcaagct ttctctaagg gctctgtaca agctgatcta actctgatgt 6240
   ttgacaattt tgaggtggac tttgtgggag cagccgtatc ttgtgatgcc gccttcttaa 6300
   atttgacagg ttgctattct tgcaatgcag gggccagggt ctgcctgtct atcacatcca 6360
   caggaactgg atccctctct gcccacaata aggatgggtc tctgcatata gtccttccat 6420
   cagagaatgg aacaaaagac cagtgtcaga tactacactt cactgtgcct gaagtagagg 6480
   aggagtttat gtactcttgt gatggagatg agcggcctct gttggtgaag gggaccctga 6540
   tagccattga tccatttgat gataggcggg aagcaggggg ggaatcaaca gttgtgaatc 6600
   caaaatctgg atcttggaat ttctttgact ggttttctgg actcatgagt tggtttggag 6660
   ggcctcttaa aactatactc ctcatttgcc tgtatgttgc attatcaatt gggctctttt 6720
   tcctccttat atatcttgga agaacaggcc tctctaaaat gtggcttgct gccactaaga 6780
   aggcctcata gatcaggaag agctctgtca agggtcaaga caattctgca gatatccagc     6840
   acagtggcgg ccgctcgagg aattctagat cccacgtcac tattgtatac tctatattat     6900
   actctatgtt atactctgta atcctactca ataaacgtgt cacgcctgtg aaaccgtact     6960
   aagtctcccg tgtcttctta tcaccatcag gtgacatcct cgcccaggct gtcaatcatg     7020
   ccggtatcga ttccagtagc accggcccca cgctgacaac ccactcttgc agcgttagca     7080
   gcgcccctct taacaagccg acccccacca gcgtcgcggt tactaacact cctctccccg     7140
   acctgcagcc caagcttggc gtaatcatgg tcatagctgt ttcctgtgtg aaattgttat     7200
   ccgctcacaa ttccacacaa catacgagcc ggaagcataa agtgtaaagc ctggggtgcc     7260
   taatgagtga gctaactcac attaattgcg ttgcgctcac tgcccgcttt ccagtcggga     7320
   aacctgtcgt gccagctgca ttaatgaatc ggccaacgcg cggggagagg cggtttgcgt     7380
   attgggcgct cttccgcttc ctcgctcact gactcgctgc gctcggtcgt tcggctgcgg     7440
   cgagcggtat cagctcactc aaaggcggta atacggttat ccacagaatc aggggataac     7500
   gcaggaaaga acatgtgagc aaaaggccag caaaaggcca ggaaccgtaa aaaggccgcg     7560
   ttgctggcgt ttttccatag gctccgcccc cctgacgagc atcacaaaaa tcgacgctca     7620
   agtcagaggt ggcgaaaccc gacaggacta taaagatacc aggcgtttcc ccctggaagc     7680
   tccctcgtgc gctctcctgt tccgaccctg ccgcttaccg gatacctgtc cgcctttctc     7740
   ccttcgggaa gcgtggcgct ttctcatagc tcacgctgta ggtatctcag ttcggtgtag     7800
   gtcgttcgct ccaagctggg ctgtgtgcac gaaccccccg ttcagcccga ccgctgcgcc     7860
   ttatccggta actatcgtct tgagtccaac ccggtaagac acgacttatc gccactggca     7920
   gcagccactg gtaacaggat tagcagagcg aggt                                 7954